# EUROPEAN PATENT SPECIFICATION

(11) **EP 4 252 852 B1**
(45) Date of publication and mention of the grant of the patent: **26.08.2026**
(21) Application number: 23176640.3
(22) Date of filing: 29.03.2019
(51) Int. Cl.: A61K 35/13, A61K 35/15, A61P 35/00, C07K 16/28, A61K 39/39, A61K 39/00, A61K 49/00, A61K 39/395, A61K 40/11, A61K 40/19, A61K 40/24, A61K 40/42

(54) **METHODS AND MATERIALS FOR TREATING CANCER BASED ON DENDRIIC CELLS PRESENTING TUMOR ANTIGENS**
VERFAHREN UND MATERIALIEN ZUR BEHANDLUNG VON KREBS UNTER VERWENDUNG VON TUMORANTIGEN-PRÄSENTIERENDEN DENDRITISCHEN ZELLEN
MÉTHODES ET MATÉRIELS POUR LE TRAITEMENT DU CANCER BASÉS SUR LES CELLULES DENDRITIQUE PRÉSENTANT DES ANTIGÈNES TUMORAUX

(30) Priority: 29.03.2018 US 201862650171 P; 26.10.2018 US 201862751334 P
(43) Date of publication of application: 04.10.2023
(62) Document number(s) of the earlier application(s) in accordance with Art. 76 EPC: 19775249.6 / 3 773 625
(73) Proprietor: Mayo Foundation for Medical Education and Research, Rochester, Minnesota 55905 (US)
(72) Inventor: EVGIN, Laura, Rochester, MN 55902-3633 (US); VILE, Richard G., Rochester, MN 55902 (US); KOTTKE, Timothy J., Oronoco, MN 55960 (US); HUFF, Amanda L., Rochester, MN 55901 (US)
(74) Representative: Fish & Richardson P.C.

(56) References cited:
- WO-A1-2016/083791
- WO-A1-2016/149045
- WO-A1-2017/004165
- WO-A1-2018/165631
- KOTTKE T ET AL: "Immunotherapy for tumours through APOBEC3B-induced neo-epitope generation in combination with immune checkpoint blockade", HUMAN GENE THERAPY 2017 MARY ANN LIEBERT INC. NLD, vol. 28, no. 12, 2017, XP055742223, ISSN: 1557-7422
- AMANDA L. HUFF ET AL: "APOBEC3 Mediates Resistance to Oncolytic Viral Therapy", MOLECULAR THERAPY - ONCOLYTICS, vol. 11, 1 December 2018 (2018-12-01), pages 1 - 13, XP055741656, ISSN: 2372-7705, DOI: 10.1016/j.omto.2018.08.003
- EVGIN L ET AL: "Cancer immunotherapy with APOBEC3B-induced heteroclitic library tumor cell vaccines and immune checkpoint blockade", MOLECULAR THERAPY 20180501 CELL PRESS NLD, vol. 26, no. 5, Supplement 1, 1 May 2018 (2018-05-01), XP055793232, ISSN: 1525-0024
- HOLLERN D P ET AL: "Apobec3 induced mutagenesis sensitizes murine models of triple negative breast cancer to immunotherapy by activating B-cells and CD4+ T-cells", CANCER RESEARCH 20190201 AMERICAN ASSOCIATION FOR CANCER RESEARCH INC. NLD, vol. 79, no. 4, Supplement 1, 1 February 2019 (2019-02-01), XP055793236, ISSN: 1538-7445
- DRISCOLL CHRISTOPHER B. ET AL: "APOBEC3B-mediated corruption of the tumor cell immunopeptidome induces heteroclitic neoepitopes for cancer immunotherapy", vol. 11, no. 1, 7 February 2020 (2020-02-07), XP055793067, Retrieved from the Internet <URL:http://www.nature.com/articles/s41467-020-14568-7> DOI: 10.1038/s41467-020-14568-7
- YU JOHN S. ET AL: "Vaccination with Tumor Lysate-Pulsed Dendritic Cells Elicits Antigen-Specific, Cytotoxic T-Cells in Patients with Malignant Glioma", vol. 64, no. 14, 15 July 2004 (2004-07-15), US, pages 4973 - 4979, XP093084044, ISSN: 0008-5472, Retrieved from the Internet <URL:https://watermark.silverchair.com/zch01404004973.pdf?token=AQECAHi208BE49Ooan9kkhW_Ercy7Dm3ZL_9Cf3qfKAc485ysgAAA24wggNqBgkqhkiG9w0BBwagggNbMIIDVwIBADCCA1AGCSqGSIb3DQEHATAeBglghkgBZQMEAS4wEQQMdIocrxoSxPRj4Dr-AgEQgIIDIYrQg08lyXozZHHn56Fojz3fqY_2a9yahEssy0Mm750dgjOLd2H8KMHJonWoxAxCUvK4dmgVhmzO5-CF70IZD> DOI: 10.1158/0008-5472.CAN-03-3505
- DANIELA ANGELA COVINO ET AL: "Understanding the regulation of APOBEC3 expression: Current evidence and much to learn", JOURNAL OF LEUKOCYTE BIOLOGY, vol. 103, no. 3, 15 December 2017 (2017-12-15), GB, pages 433 - 444, XP055754345, ISSN: 0741-5400, DOI: 10.1002/JLB.2MR0717-310R
- DATABASE BIOSIS [online] BIOSCIENCES INFORMATION SERVICE, PHILADELPHIA, PA, US; 2 December 2016 (2016-12-02), YAMAZAKI HIROYUKI ET AL: "Aberrantly Expressed APOBEC3B Induces Mutations in Multiple Myeloma", Database accession no. PREV201700342898
- BLOOD, vol. 128, no. 22, 2 December 2016 (2016-12-02), 58TH ANNUAL MEETING AND EXPOSITION OF THE AMERICAN-SOCIETY-OF-HEMATOLOGY (ASH); SAN DIEGO, CA, USA; DECEMBER 03 -06, 2016, pages 4453, ISSN: 0006-4971(print)
- DATABASE EMBASE [online] ELSEVIER SCIENCE PUBLISHERS, AMSTERDAM, NL; 1 February 2019 (2019-02-01), HOLLERN D P ET AL: "Apobec3 induced mutagenesis sensitizes murine models of triple negative breast cancer to immunotherapy by activating B-cells and CD4+ T-cells", Database accession no. EMB-627250658
- HOLLERN D P ET AL: "Apobec3 induced mutagenesis sensitizes murine models of triple negative breast cancer to immunotherapy by activating B-cells and CD4+ T-cells", CANCER RESEARCH 20190201 AMERICAN ASSOCIATION FOR CANCER RESEARCH INC. NLD, vol. 79, no. 4, Supplement 1, 1 February 2019 (2019-02-01), ISSN: 1538-7445
- DATABASE EMBASE [online] ELSEVIER SCIENCE PUBLISHERS, AMSTERDAM, NL; 2017, KOTTKE T ET AL: "Immunotherapy for tumours through APOBEC3B-induced neo-epitope generation in combination with immune checkpoint blockade", Database accession no. EMB-620362456
- KOTTKE T ET AL: "Immunotherapy for tumours through APOBEC3B-induced neo-epitope generation in combination with immune checkpoint blockade", HUMAN GENE THERAPY 2017 MARY ANN LIEBERT INC. NLD, vol. 28, no. 12, 2017, pages A16 CONF 20171017 to 20171020 Berlin - 25th Anni, ISSN: 1557-7422
- DATABASE EMBASE [online] ELSEVIER SCIENCE PUBLISHERS, AMSTERDAM, NL; 1 May 2018 (2018-05-01), EVGIN L ET AL: "Cancer immunotherapy with APOBEC3B-induced heteroclitic library tumor cell vaccines and immune checkpoint blockade", Database accession no. EMB-623339154
- EVGIN L ET AL: "Cancer immunotherapy with APOBEC3B-induced heteroclitic library tumor cell vaccines and immune checkpoint blockade", MOLECULAR THERAPY 20180501 CELL PRESS NLD, vol. 26, no. 5, Supplement 1, 1 May 2018 (2018-05-01), pages 442 CONF 20180516 to 20180519 Chicago, IL - 21st Annu, ISSN: 1525-0024

## Description

### CROSS-REFERENCE TO RELATED APPLICATIONS

This application claims the benefit of U.S. Patent Application Serial No. 62/650,171, filed on March 29, 2018, and of U.S. Patent Application Serial No. 62/751,334, filed on October 26, 2018.

### SEQUENCE LISTING

The instant application contains a Sequence Listing which has been submitted electronically in ASCII format. Said ASCII copy, created on March 29, 2019, is named 07039-1786WO1_SL.txt and is 164,895 bytes in size.

### BACKGROUND

### 1. Technical Field

This document relates to materials for treating a mammal having cancer. For example, compositions *(e.g.,* vaccines) containing cells *(e.g.,* tumor cells) expressing APOBEC3 can be administered to a mammal (*e.g*., a human) having cancer to induce an immune response (*e.g*., an anti-tumor immune response) within the mammal.

### 2. Background Information

Pediatric high grade gliomas (pHGGs) including anaplastic astrocytoma, glioblastoma multiforme (GBM), and diffuse intrinsic pontine glioma (DIPG), manifest as an aggressive spectrum of diseases that represent a devastating and urgent unmet clinical need. Although relatively rare in children (1.78 per 100 000), patients are faced with a dismal prognosis with a two year median survival ranging from 30% for cerebral cortex located tumors, to less than 10% for pontine located tumors (Jones, Nat Rev Cancer 14, doi:10.1038/nrc3811 (2014); and Mackay et al. Cancer Cell 32:520-537 (2017)).

Kottke T et al. describe in Human Gene Therapy, 2017, vol. 28, no. 12, an immunotherapy for tumours through APOBEC3B-induced neo-epitope generation in combination with immune checkpoint blockade.

WO 2017/004165 A1 describes a method of treating a subject having a tumor that includes administering to the subject an amount of an APOBEC3 upregulator effective to increase mutagenesis in cells of the tumor.

WO 2016/149045 A1 describes methods of using recombinant galectin proteins to reverse HIV latency.

Yu John S. et al. describe in Cancer Research, vol. 64, no. 14, 15 July 2004, pages 4973 - 4979, a vaccination with Tumor Lysate-Pulsed Dendritic Cells Elicits Antigen-Specific, Cytotoxic T-Cells in Patients with Malignant Glioma.

WO 2016/083791 A1 describes a method of providing a prognosis in a cancer patient comprising analysing a tumour image to calculate a metric of immune infiltration for the tumour, and a method of analysing a tumour image.

### SUMMARY

The present invention is defined by the independent claims. The dependent claims depict additional embodiments of the invention.

This document provides materials related to treating a mammal having cancer. For example, this document provides compositions *(e.g.,* vaccines) containing cells *(e.g.,* tumor cells) expressing probable DNA dC->dU-editing enzyme (APOBEC3) polypeptides. The compositions *(e.g.,* vaccines) provided herein can be administered to a mammal (*e.g*., a human) having cancer to induce an immune response (*e.g*., an anti-tumor immune response) within the mammal.

As demonstrated herein, tumor cells overexpressing an APOBEC3B polypeptide generate a library of heteroclictic epitopes which prime both CD4 and CD8 T cells that have escaped central tolerance and which recognize newly mutated antigens from the vaccine. These T cells generated against the APOBEC3B-induced somatic mutations in the tumor cell vaccine were able to cross react with unaltered self-epitopes expressed on the tumor cells. As also demonstrated herein, the acquired mutational burden on APOBEC3B overexpressing melanoma tumor cells included neoantigens (*e.g*., tumor-specific antigens) having epitopes with a MHC class I higher binding affinity than the wild type antigens. These results demonstrate that *ex vivo* production of a tumor antigen loaded autologous DC can facilitate T cell priming *in situ.* The ability to induce these neoantigens provides an opportunity to generate a CD8 T cell response and/or a CD4 T cell response that cross-reacts with tumor cells to mediate anti-tumor therapy *(e.g.,* an anti-tumor T cell response).

In general, one aspect of this document features a composition that includes one or more tumor cells that include a viral vector encoding APOBEC3B polypeptides loaded into or fed to a dendritic cell. The viral vector is an adenoviral vector, and a retroviral vector. The one or more tumor cells express the APOBEC3B polypeptides and generate a library of mutated tumor antigens within the tumor cells. The tumor antigens include one or more heteroclitic mutations. The dendritic cell presents the library of mutated tumor antigens. The APOBEC3B polypeptides can be human APOBEC3B polypeptides. The tumor antigens can include one or more self antigens. The tumor antigens can include one or more non-self antigens. The tumor antigens can include one or more neoantigens. One or more tumor cells can include autologous cells. One or more tumor cells can include allogeneic cells.

In another aspect, this document provides the composition described above for use in a method for treating a cancer in a mammal. The method includes administering a composition to a mammal. One or more tumor cells can be obtained from the mammal to be treated. One or more tumor cells can be obtained from a donor mammal. The mammal can be a human. The cancer can be a glioma, a melanoma, a prostate cancer, and lung cancer.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the resent specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

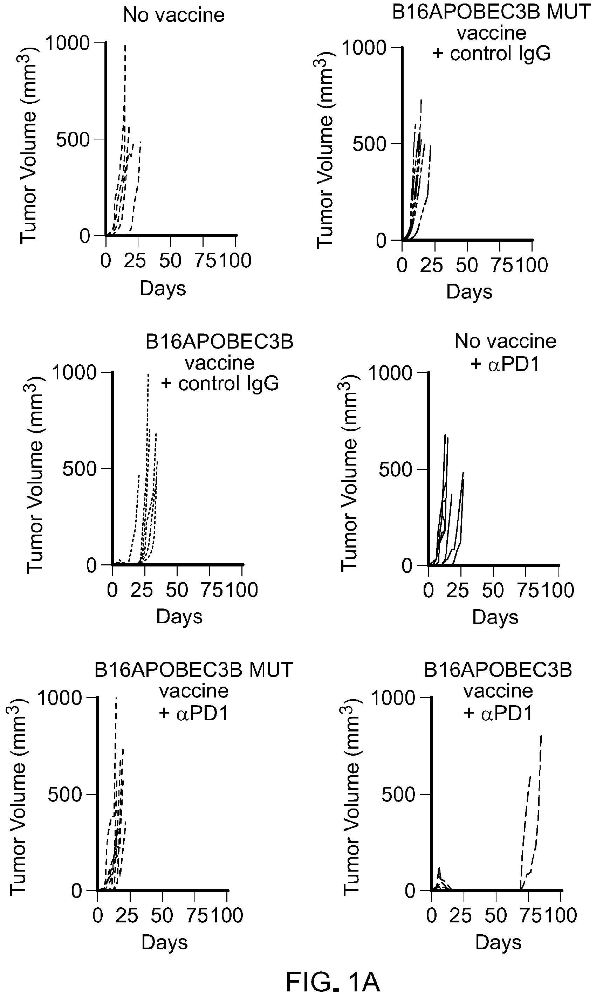

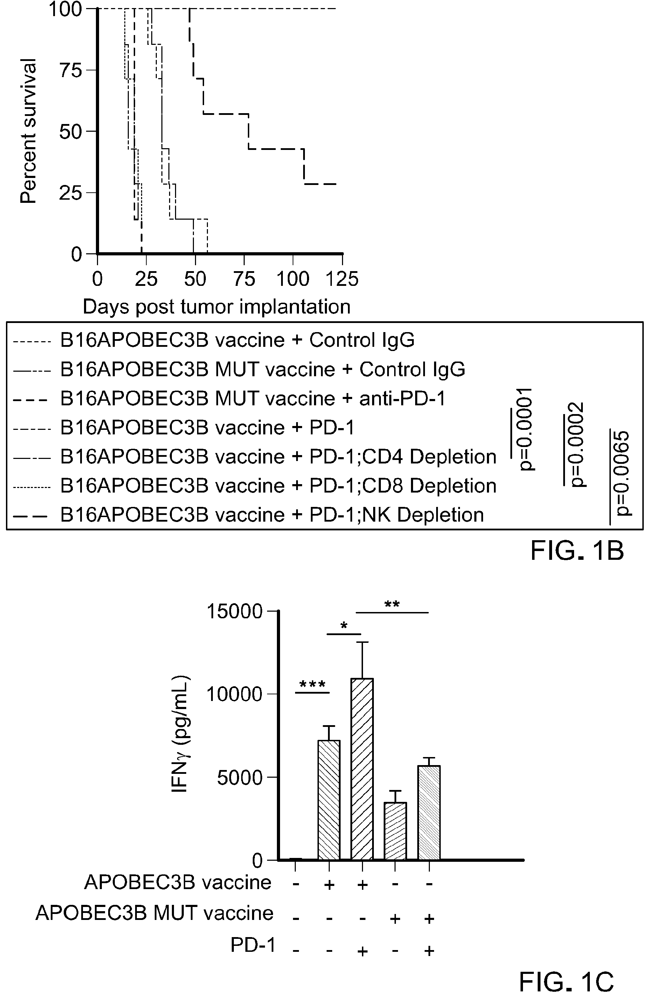

Figure 1. (A) C57BL/6 bearing subcutaneous B16 tumors were treated with freeze-thawed B16APOBEC3B or B16APOBEC3B MUT vaccine for 5 consecutive days beginning on day 5. Anti-PD1 was administered 3 times per week for two weeks beginning on day 12. (B) As described in A with CD8, CD4 or NK depletion concurrent with anti-PD1. (C) In vitro splenocyte cytokine recall from mice treated in A and co-cultured with parental B16 cells.

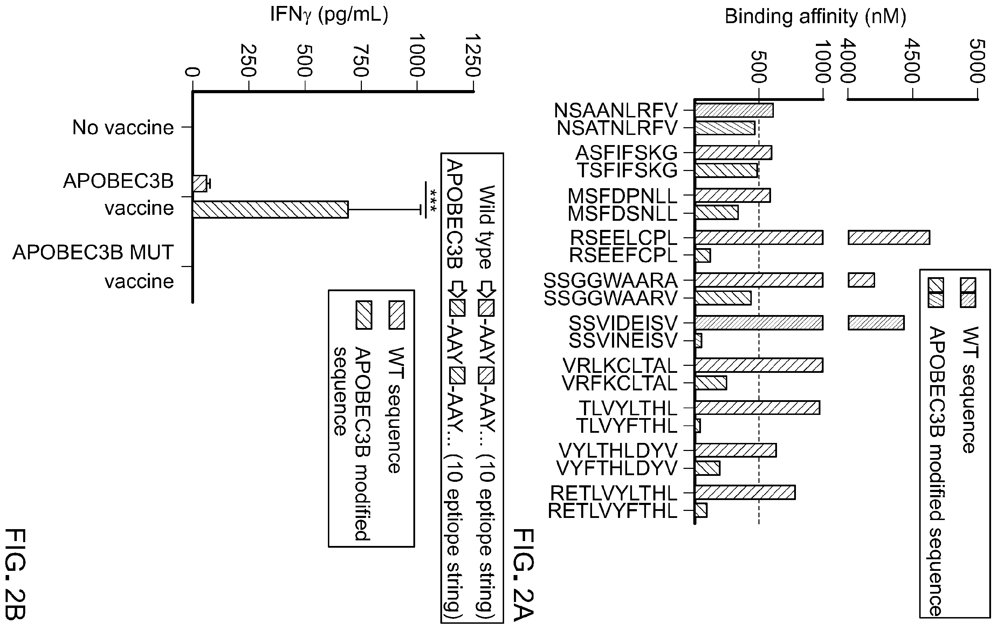

Figure 2. (A) Predicted binding affinity to H2K^{b} (darker shades) or H2D^{b} (lighter shades) of peptides encoding non-synonymous mutations identified from the B16APOBEC3B overexpressing cell line using NetMHC4.0. Bolded letter indicates mutation. Figure 2A discloses SEQ ID NOS 3-22, respectively, in order of appearance. (B) Splenocytes from mice vaccinated with five doses of the B16APOBEC3B or B16APOBEC3BMUT vaccine were co-cultured with B16 cells transfected with a polyepitope construct expressing the wild type or the APOBEC3B mutations identified from A.

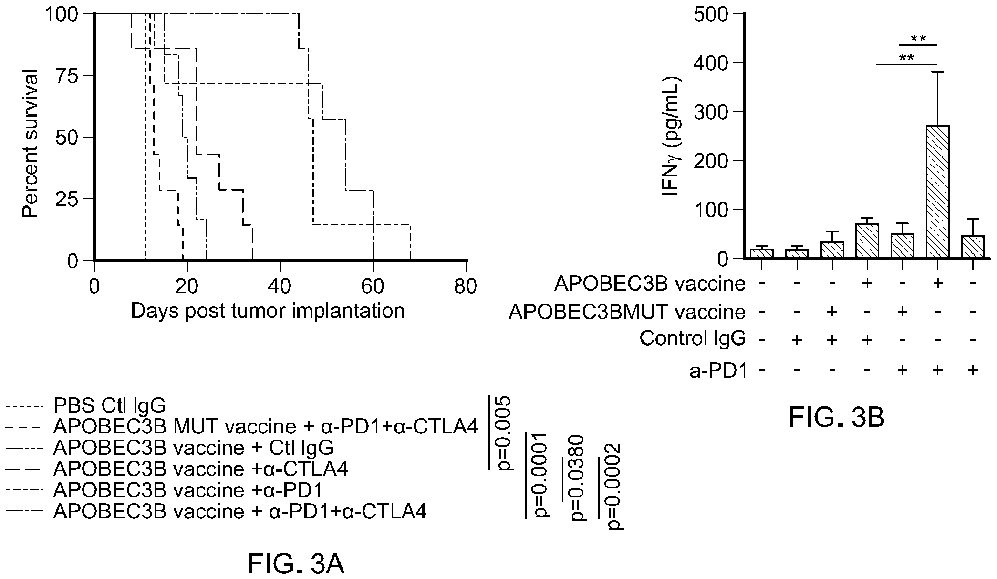

Figure 3. (A) GL261 cells were implanted into C57BL/6 mice and treated with a freeze thawed APOEBC3B or APOBEC3B MUT modified GL261 cell vaccine for 5 consecutive days beginning on day 5. Immune checkpoint inhibitors were administered 3 times per week for 2 weeks beginning on day 5. (B) Splenocytes from mice treated as in A were co-cultured with parental GL261 cells and IFNy was measured by ELISA. No additional checkpoint inhibitors were included in the in vitro assay.

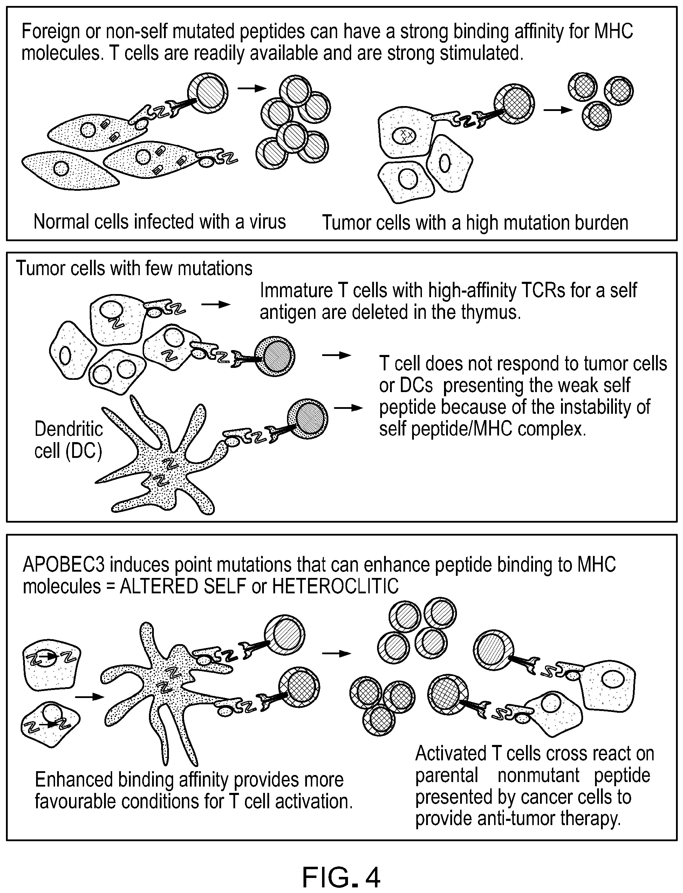

Figure 4. Schematic representation of the APOBEC3B- modified altered self tumor cell vaccination approach.

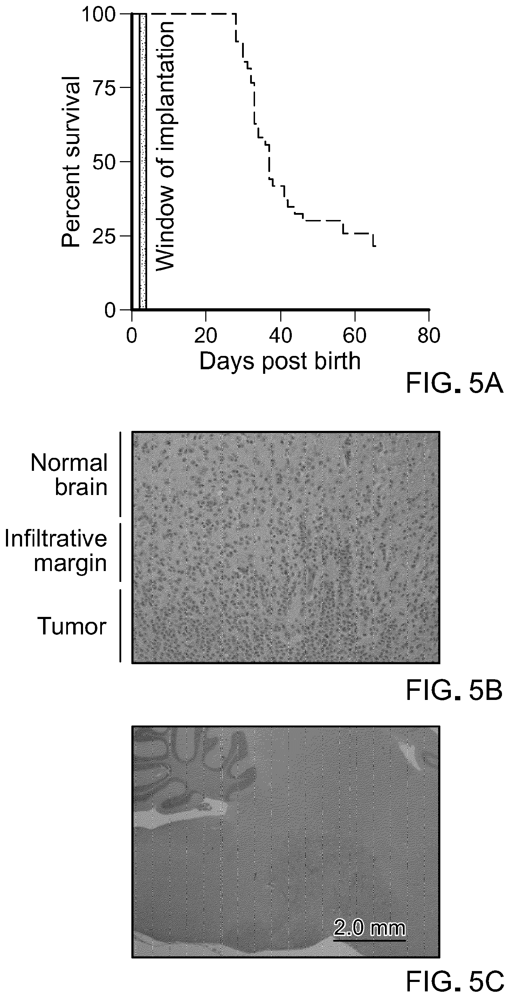

Figure 5. RCAS DIPG genetically engineered mouse model. DF-1 producer cells are injected into the brainstem of Nestin tv-a/p53 floxed neonatal mice (Postnatal day 3-4). Viruses encoding PDGFB, Cre, and, H3.3 K27M infect Nestin expressing cells. Tumor symptoms develop within 4-6 weeks (A). H&E reveals that tumors exhibit high-grade characteristics and infiltrate adjacent brain parenchyma (B). Tumors grow out of midbrain and pons structures (C).

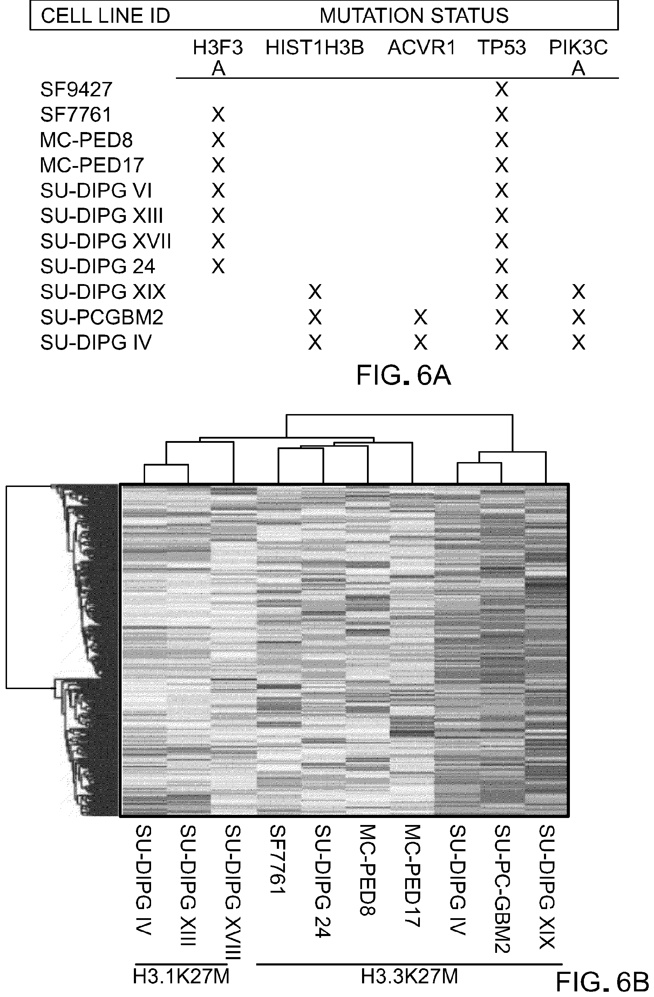

Figure 6. (A) Mutational characterization of human DIPG cell lines. (B) Unsupervised hierarchy clustering and heatmap of genes with a differential expression between cell lines bearing an H3.1 or H3.3 K27M mutation.

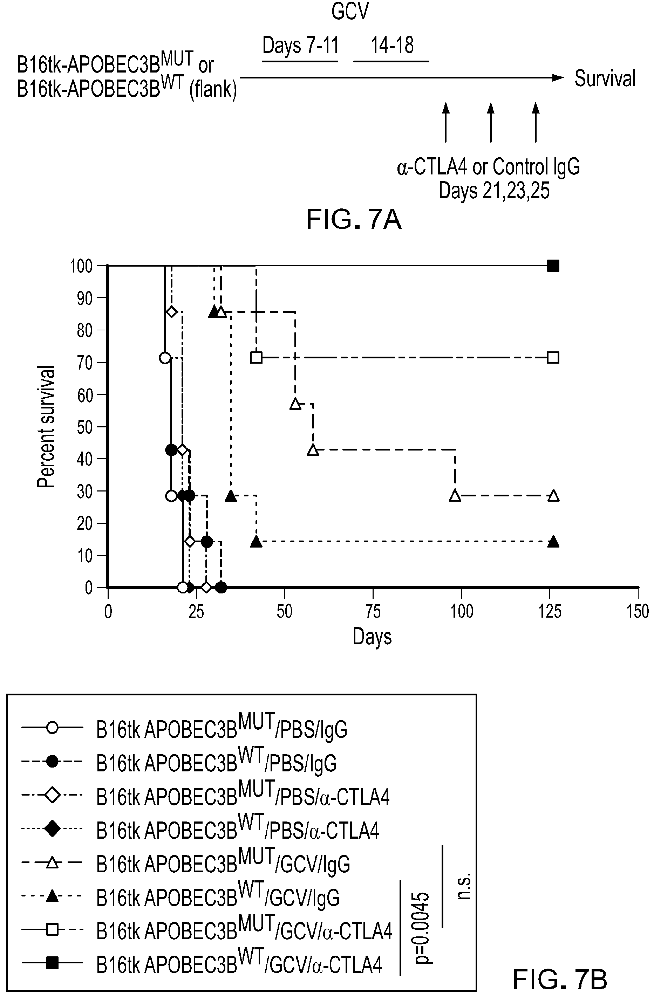

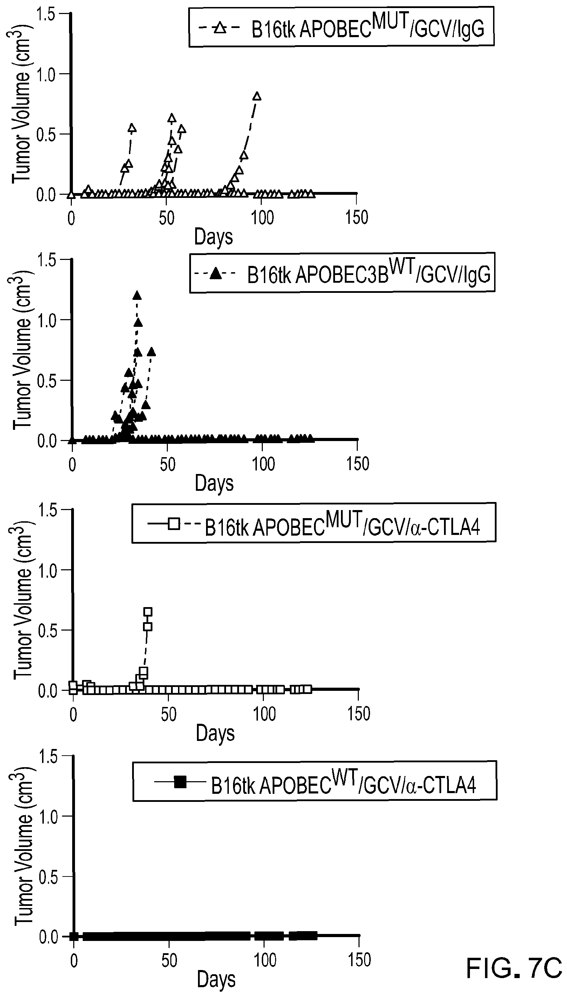

Figure 7. APOBEC3B expression enhances tumor escape but sensitizes tumors to immune checkpoint blockade therapy. (A) On day 0, 2x10⁵ B16TK cells expressing either APOBEC3B^{MUT} or APOBEC3B^{WT} were subcutaneously implanted into the right flank of C57Bl/6 mice. Two 5-day courses of GCV therapy (50 mg/kg i.p.) were administered from days 7-11, and 14-18, followed by anti-CTLA4 antibody or control IgG (5 mg/kg i.p.) on days 21, 23, and 25. (B, C) Mice were treated according to the regimen in (A) (n=7 mice/group) tumor volumes were measured three times per week and sacrificed when tumors grew above 1 cm in length or width.

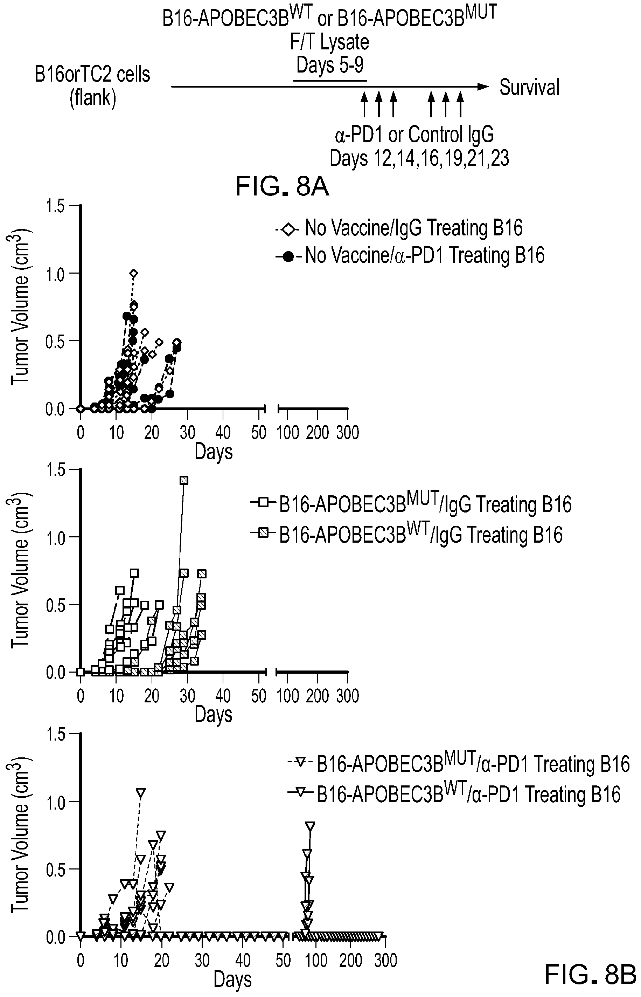

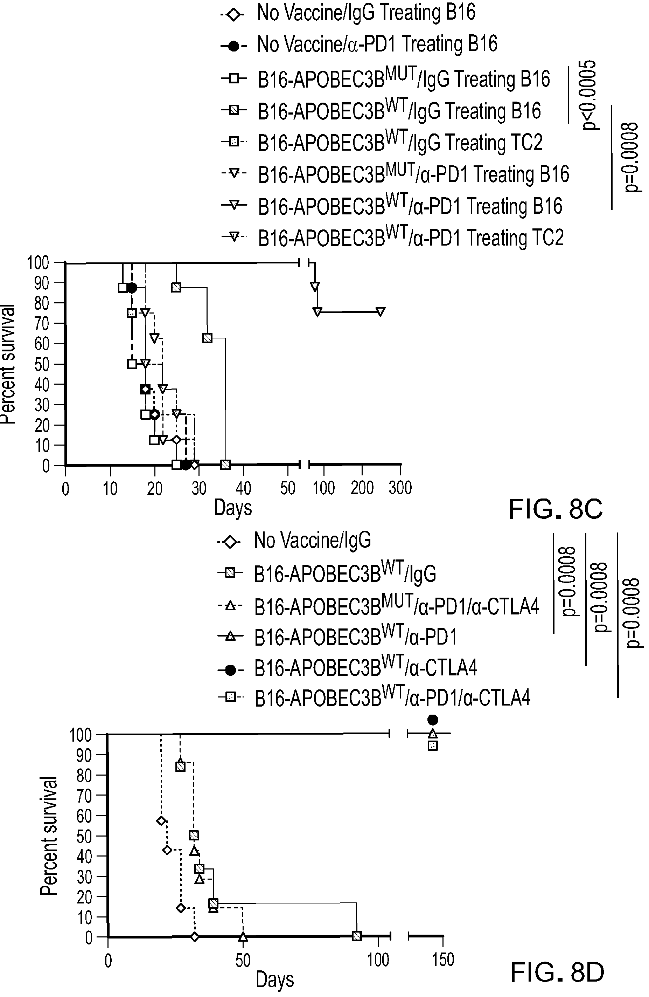

Figure 8. APOBEC3B enhances B16 cell lysate vaccine and synergizes with immune checkpoint blockade therapy. (A) On day 0, 2x10⁵ B16 murine melanoma or TC2 murine prostate carcinoma were implanted subcutaneously into the right flank of C57B1/6 mice. One 5-day course of APOBEC3B^{WT} or APOBEC3B^{MUT}-modified B16 cell vaccines (freeze/thaw lysate of 10⁶ cells i.p.) was administered from days 5-9. This was followed by anti-PD1 antibody or IgG control (12.5 mg/kg i.p.) on days 12, 14, 16, 19, 21 and 23. (B) Mice (n=8/group) were treated according to the regimen in (A) and tumor volumes were measured three times per week. (C) Kaplan-Meier survival curves representing experiment described by (A) and (B). (D) Treatment of subcutaneous B16 murine melanomas as in (A), except with the addition of anti-CTLA4 antibody (5 mg/kg i.p.) as a monotherapy or in combination with anti-PD1 (n=7 mice/group). The Black triangle, red circle, and brown square symbols represent three separate groups of 7 mice treated with B16-APOBEC3B^{WT} vaccine with anti-PD1, anti-CTLA4, or anti-PD1/anti-CTLA4 antibody therapy that all had 100 percent survival at 150 days.

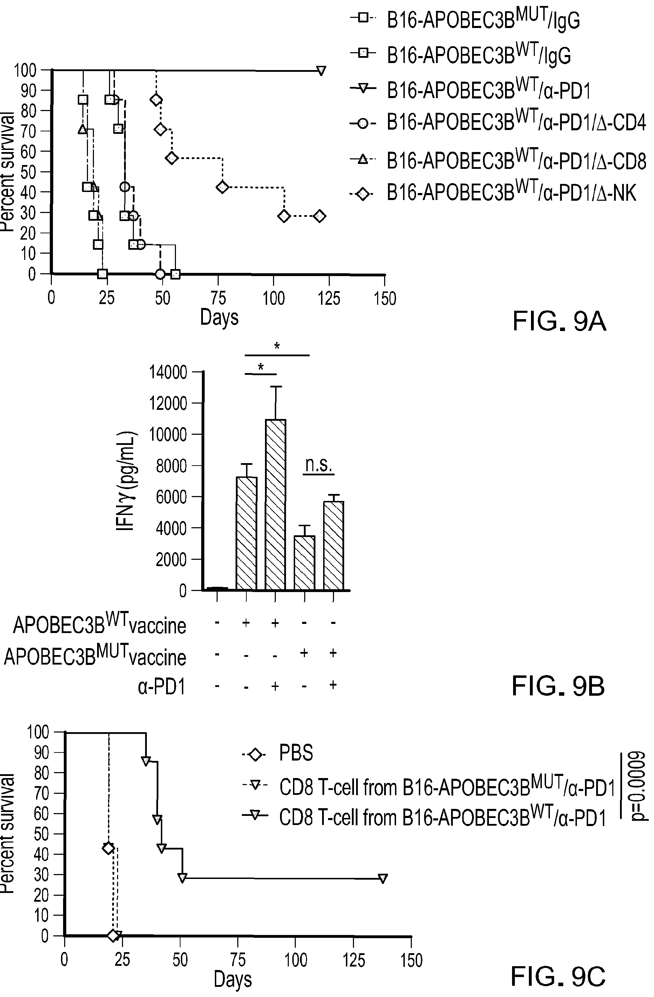

Figure 9. CD4, CD8, and NK cells mediate APOBEC3B^{WT} vaccination. (A) B16-APOBEC3B^{WT} vaccination and/or antibody-mediated checkpoint inhibition was performed as in Figure 8A, with the addition of antibodies depleting CD4 T cells, CD8 T cells, NK cells, or control IgG on day 4 after subcutaneous tumor implantation and weekly thereafter (n=7 mice/ group). (B) Spleens and lymph nodes obtained from mice (n=3 per group) succumbing to their disease in (Figure 8B, C) were made into single-cell suspensions and cocultured with B16 target cells for 72 hours. Supernatant from the co-culture was assayed using a mouse interferon gamma ELISA. ANOVA used followed by Tukey's multiple comparison test. * p≤0.05. (C) Spleens and lymph nodes obtained from mice treated with B16-APOBEC3B^{WT} vaccination and/or antibody-mediated checkpoint inhibition were made into single-cell suspensions and reinfused at dose of 1.2x10⁷ cells intravenously per B16 subcutaneous tumor-bearing mouse (n=7 mice per group).

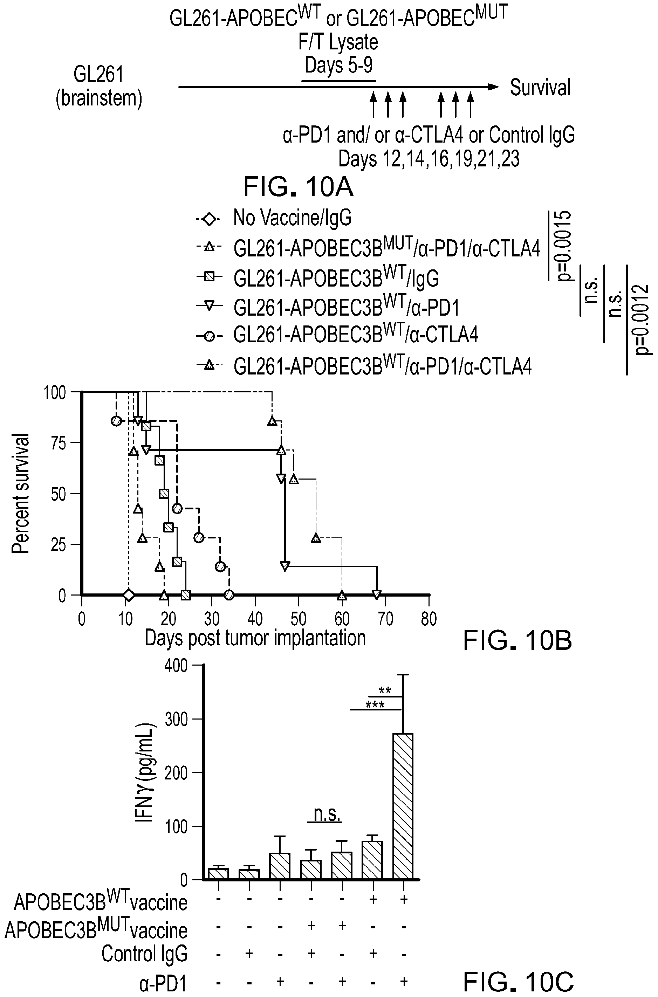

Figure 10. APOBEC3B modified GL261 cell lysate vaccine also effective against orthotopic gliomas. (A) Treatment strategy from (Figure 8A) was adapted to target GL261 murine gliomas. In summary, on day 0, 5x10⁴ GL261 murine glioma cells were implanted into the brainstem of C57Bl/6 mice. One 5-day course of APOBEC3B^{WT} or APOBEC3B^{MUT}-modified GL261 cell vaccines (freeze/thaw lysate of 10⁶ cells i.p.) was administered from days 5-9. This was followed by anti-PD1 antibody, anti-CTLA4 antibody or IgG control (12.5 mg/kg i.p.) on days 12, 14, 16, 19, 21 and 23. (B) Kaplan-Meier survival curves representing experiment described by (A) (n=7 mice/ group). (C) Spleens and lymph nodes obtained from mice treated with GL261-APOBEC3B^{WT} vaccination and/or antibody-mediated checkpoint inhibition were made into single-cell suspensions and cocultured with GL261 target cells for 72 hours. Supernatant from the co-culture was assayed using a mouse interferon gamma ELISA. ANOVA was used followed by Tukey's multiple comparison test. ** p≤0.01, *** p≤0.001.

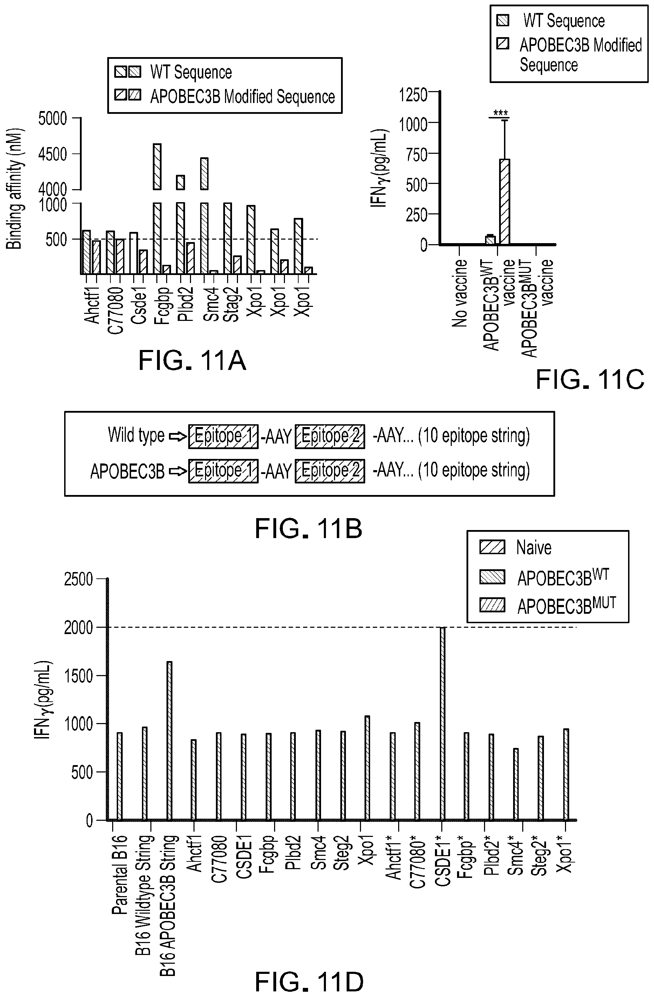

Figure 11. Mutated peptides with differential MHC binding were identified as potential heteroclitic neoepitopes. (A) The seven genes leading to ten peptides that showed differential MHC I binding between the wildtype and mutated peptide are shown. The threshold for binding affinity was set at 500 nM with weak binding being above 500 nM and strong binding being below 500 nM. (B) Expression plasmids were constructed that encoded either all ten wild type (wild type epitope string) or all ten mutated peptide sequences (APOBEC3B epitope string) with AAY spacers between each peptide to enhance cleavage and presentation. (C) Splenocytes from naïve, B16-APOBEC3B^{WT}, or B16-APOBEC3B^{MUT} vaccinated mice were collected and co-cultured with parental tumors that over-expressed either the wild type or APOBEC3B epitope string. ANOVA was used followed by Tukey's multiple comparison test. *** p≤0.001. (D) IFN-γ recall responses against (1) parental tumors, (2) wild type string, (3) APOBEC3B string, (4-11) individual wildtype epitopes, (12-19) individual mutated epitopes from T cells isolated from naïve (red), B16-APOBEC3B^{WT} (grey) or B16-APOBEC3B^{MUT} vaccinated mice (blue). Dashed line indicates upper limit of detection.

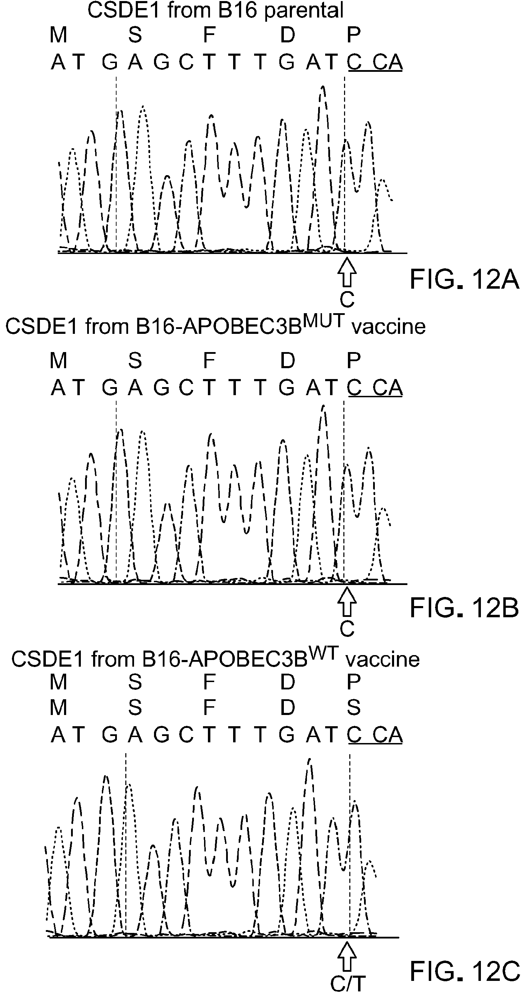

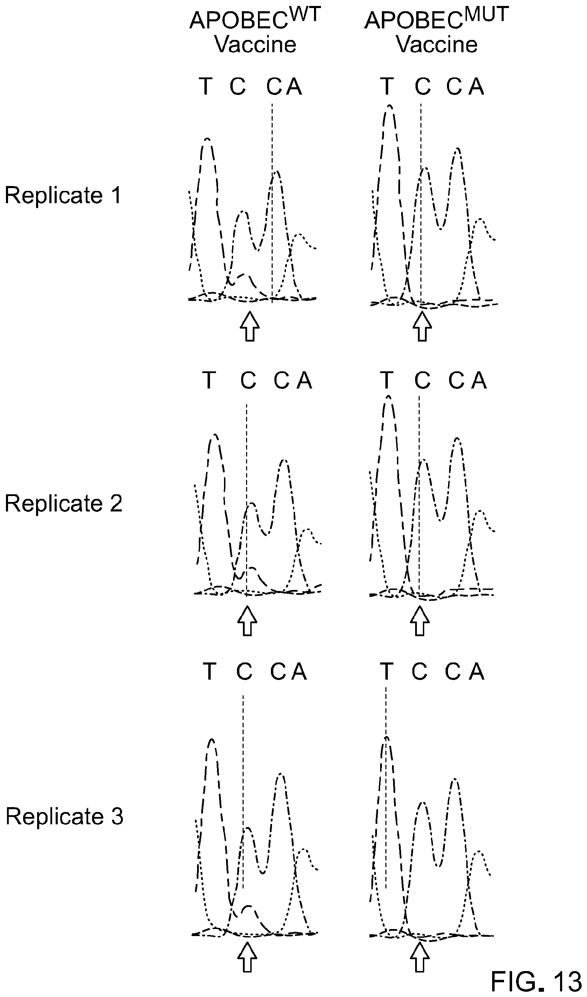

Figure 12. Sequencing of APOBEC3B^{WT} modified vaccines generates reproducible mutations in CSDE1. Sanger sequencing of CSDE1 from parental B16 cells (A), B16 cells treated with APOBEC3B^{MUT} modified vaccine (B), and B16 cells treated with APOBEC3B^{WT} vaccine (C) was performed. CSDE1 from parental B16 cells contained a wild type ATGAGCTTTGATCCA (SEQ ID NO:1) nucleic acid sequence encoding a wild type MSFDP (SEQ ID NO:23) polypeptide sequence (A), CSDE1 from B16 cells treated with APOBEC3B^{MUT} modified vaccine contained a wild type ATGAGCTTTGATCCA (SEQ ID NO:1) nucleic acid sequence encoding a wild type MSFDP (SEQ ID NO:23) polypeptide sequence (B), and CSDE1 from B16 cells treated with APOBEC3B^{WT} vaccine converted the wild type ATGAGCTTTGATCCA (SEQ ID NO:1) nucleic acid sequence encoding a wild type MSFDP (SEQ ID NO:23) polypeptide sequence to a mutated ATGAGCTTTGATTCA (SEQ ID NO:2) nucleic acid sequence encoding a mutated MSFDS (SEQ ID NO:24) polypeptide sequence (C). Figures are representative of three independent experiments. Each preparation of the APOBEC3B^{WT} modified vaccine had proportions of cells containing a C or a T at the thirteenth base pair, corresponding to the P5S amino acid change seen in Fig. 11 and Fig. 13.
Figure 13. Detection of CSDE1 mutation in vaccine preparations. Sanger sequencing of CSDE1 from B16-APOBEC3B^{WT} or B16-APOBEC3B^{MUT} vaccine preparations was performed in three independent experiments. Each preparation of the B16-APOBEC3B^{WT} vaccine had a proportion of the cells containing a C with another containing a T at the thirteenth base pair, corresponding to the P5S amino acid change.

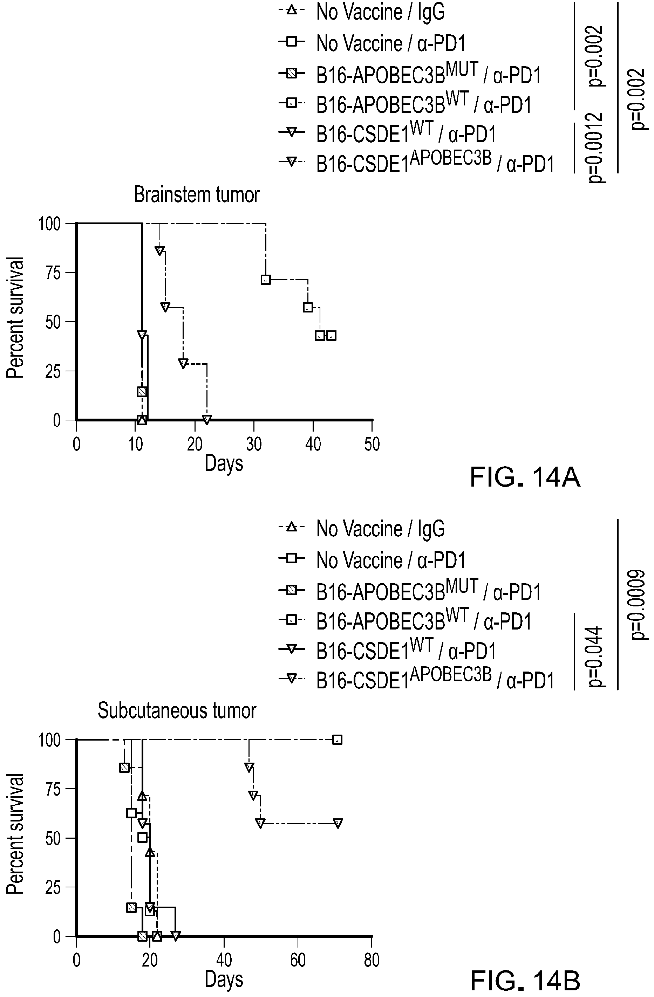

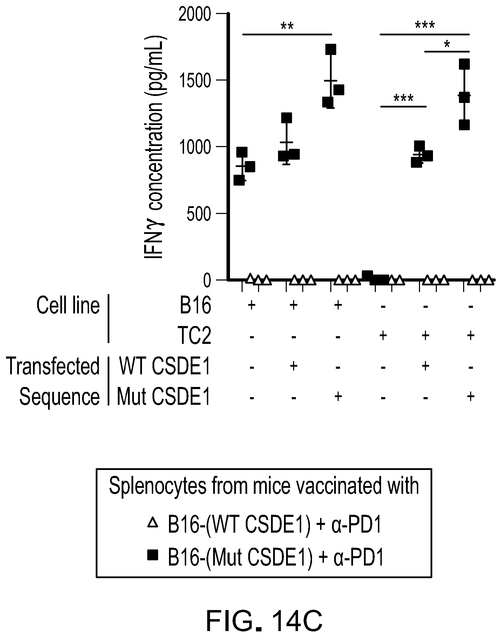

Figure 14. Vaccination with a cold shock domain-containing protein E1 (CSDE1) mutant peptide increases survival. (A) On day 0, 2x10⁵ B16 murine melanoma cells were implanted subcutaneously into the right flank of C57B1/6 mice. Two 5-day courses of B16-APOBEC3B^{WT} , B16-APOBEC3B^{MUT}, B16-CSDE1^{WT}, or B16-CSDE1^{MUT} vaccines (freeze/thaw lysate of 1x10⁶ cells i.p.) were administered from days 5-9 and 12-16. This was followed by anti-PD1 antibody or IgG control (12.5 mg/kg i.p.) on days 12-16, 19, 21, and 23. Kaplan-Meier survival curves representing experiment described. (B) On day 0, 5x10⁴ B16 murine melanoma cells were implanted into the brainstem of C57Bl/6 mice. One 5-day course of APOBEC3B^{WT} or APOBEC3B^{MUT}-modified B16 cell vaccines (freeze/thaw lysate of 10⁶ cells i.p.) was administered from days 5-9. This was followed by anti-PD1 antibody or IgG control (12.5 mg/kg i.p.) on days 12, 14, 16, 19, 21 and 23. Kaplan-Meier survival curves representing experiment described (n=7 mice/ group). (C) Spleens and lymph nodes obtained from mice treated with B16-CSDE1^{APOBEC3B} vaccination and antibody-mediated checkpoint inhibition in (B) were made into single-cell suspensions and co-cultured with B16 or TC2 target cells expressing WT-CSDE1 or Mut-CSDE1 for 72 hours. Supernatant from the co-culture was assayed using a mouse interferon gamma ELISA. ANOVA was used followed by Tukey's multiple comparison test. ** p≤0.01, *** p≤0.001.

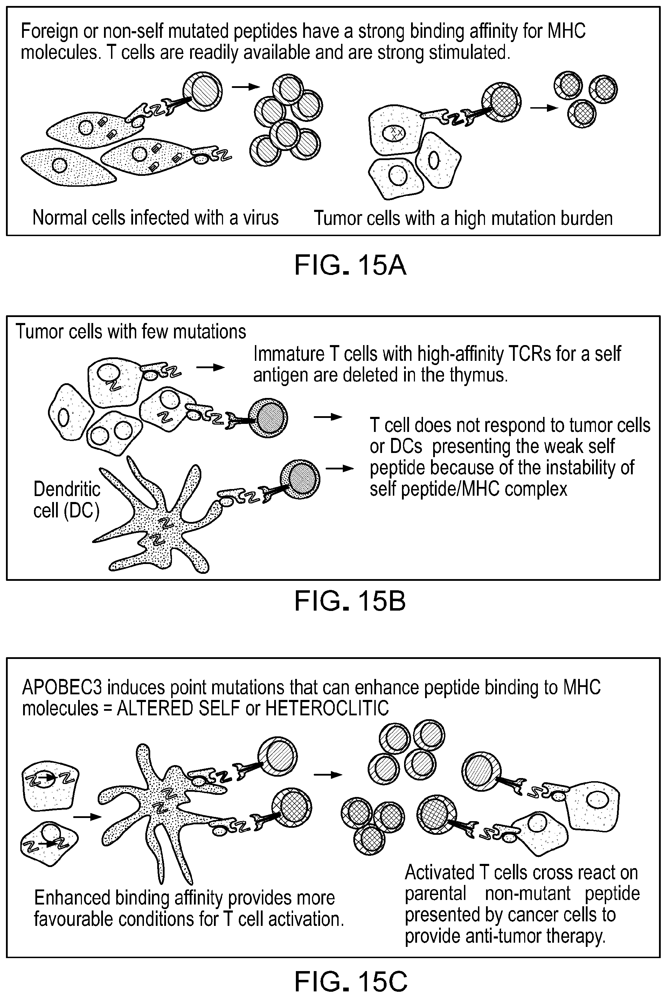

Figure 15. Model for mechanism of action of APOBEC3B modified vaccines. (A) Strong T cell responses occur when T cells recognize foreign antigen presented in MHC. If a tumor has a high mutational burden, self peptides can be recognized as non-self and the tumor will be cleared through normal immunosurveillance. (B) T cells that react strongly against self-peptides are deleted within the thymus while T cells with weak affinity are able to escape deletion. (C) Modifying tumor cell vaccines with overexpression of APOBEC3B can generate single amino acid changes throughout the genome. When these mutated peptides are presented they have the ability to activate weakly reactive T cells. These activated T cells can react against and clear non-mutated tumor.

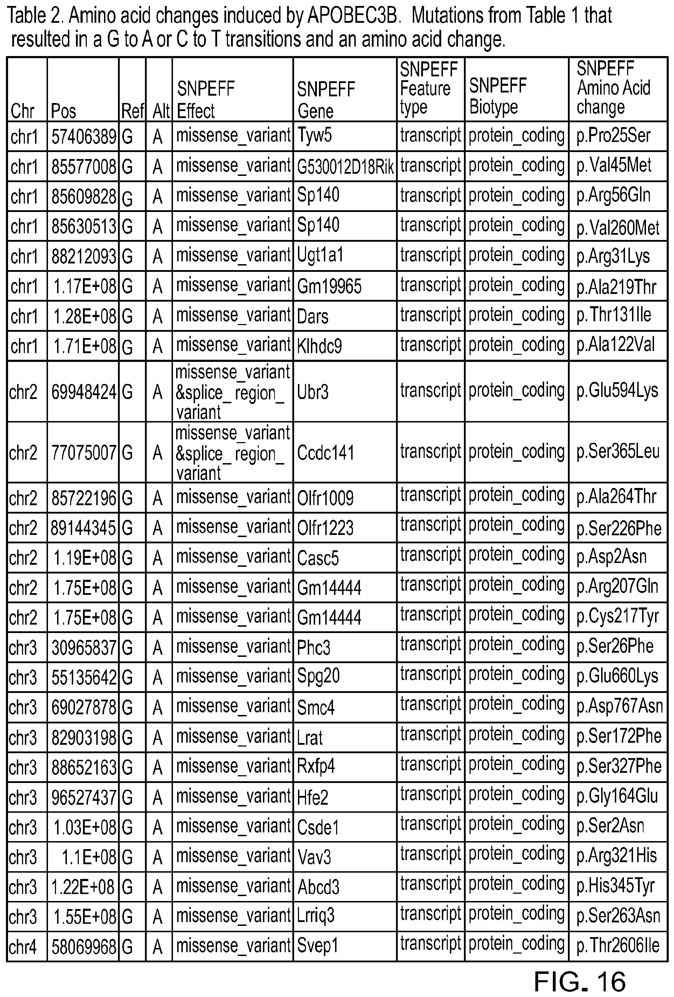

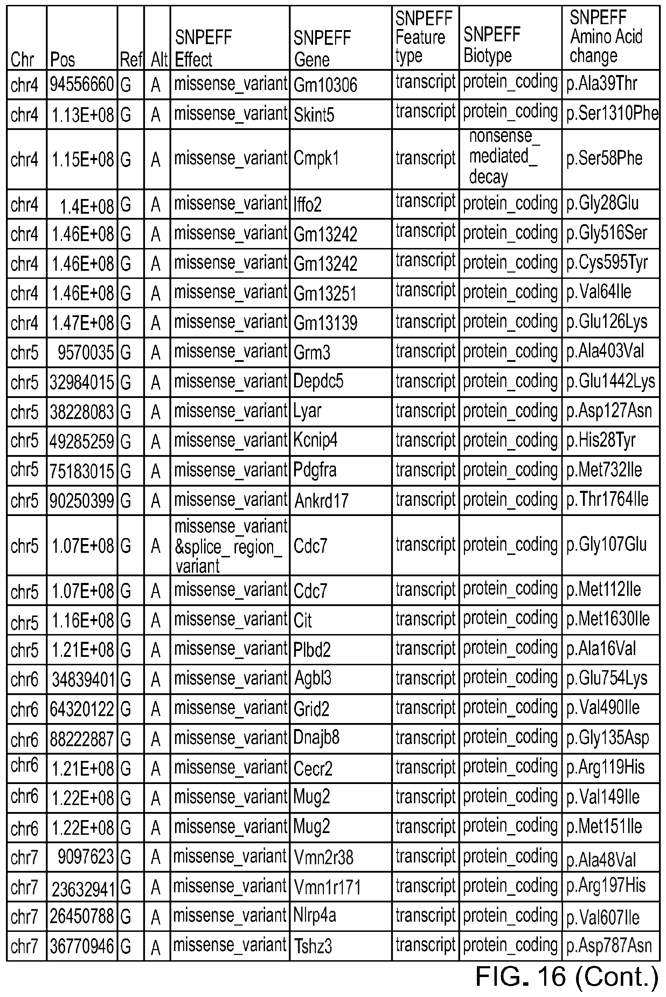

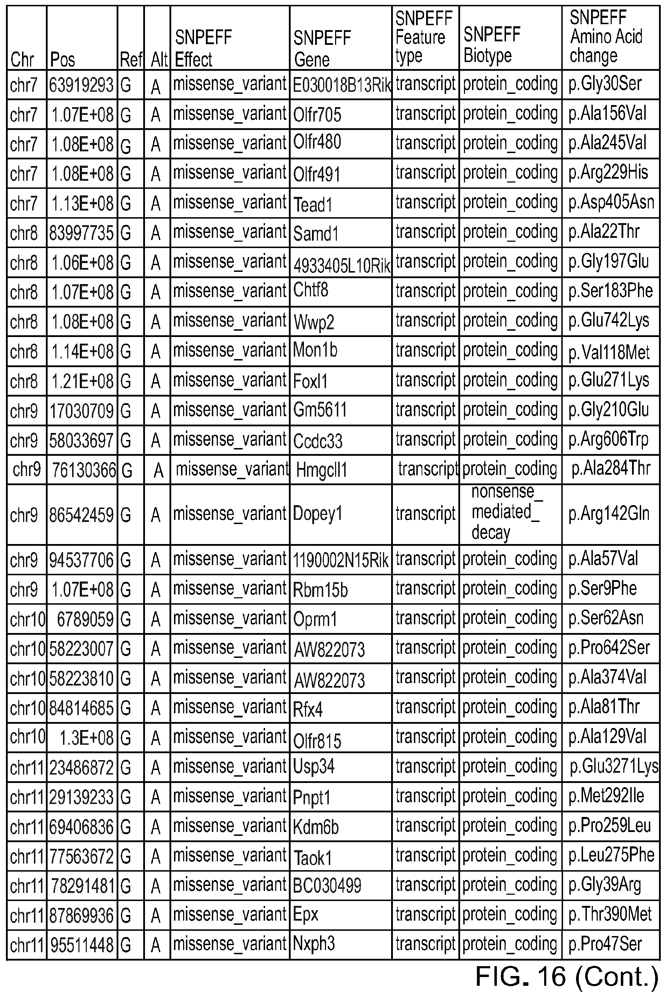

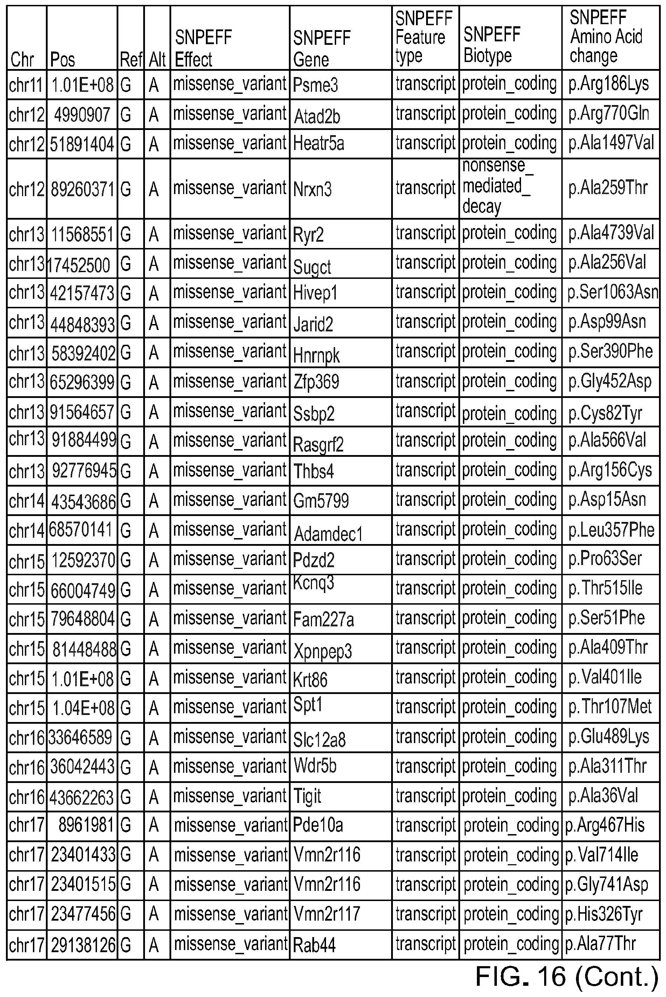

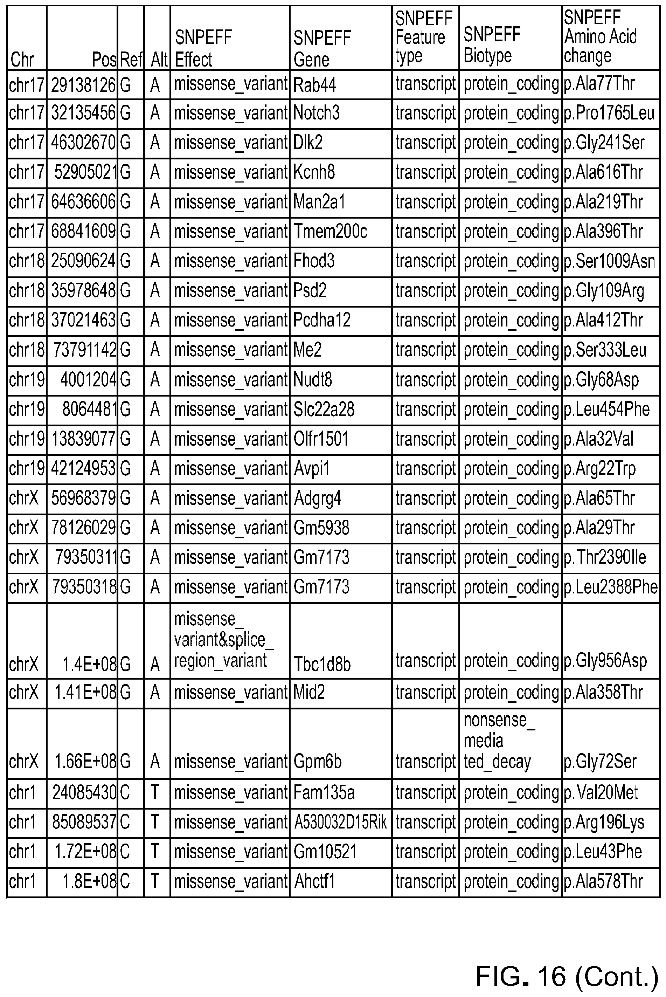

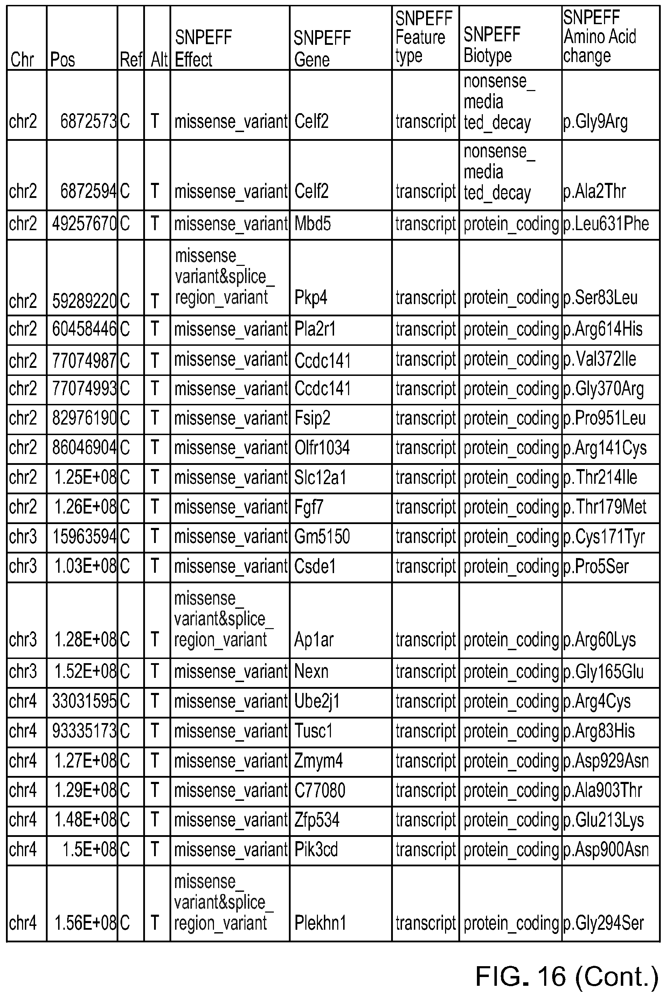

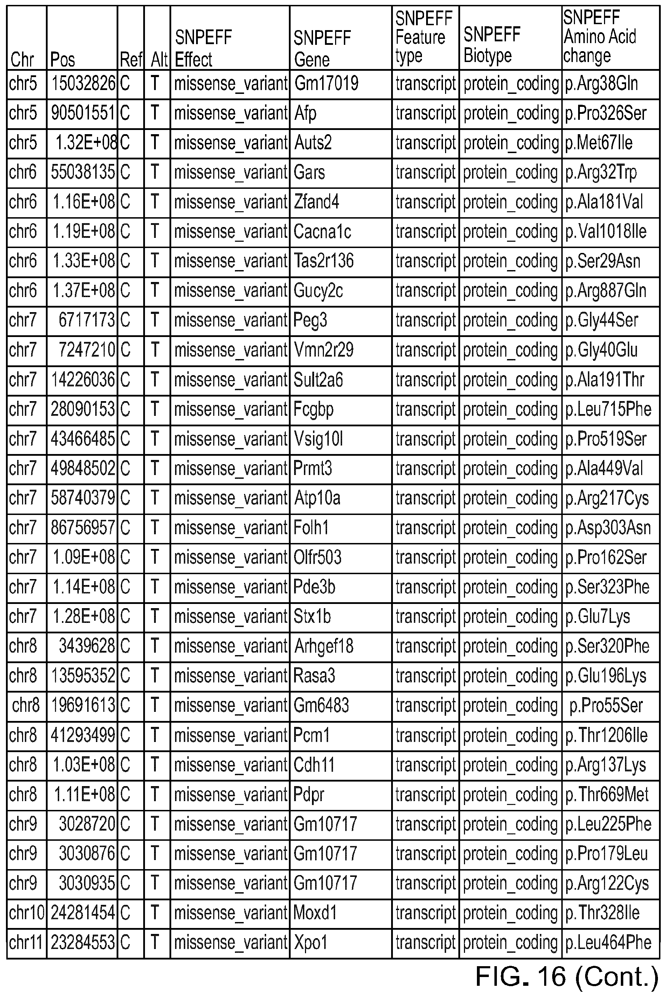

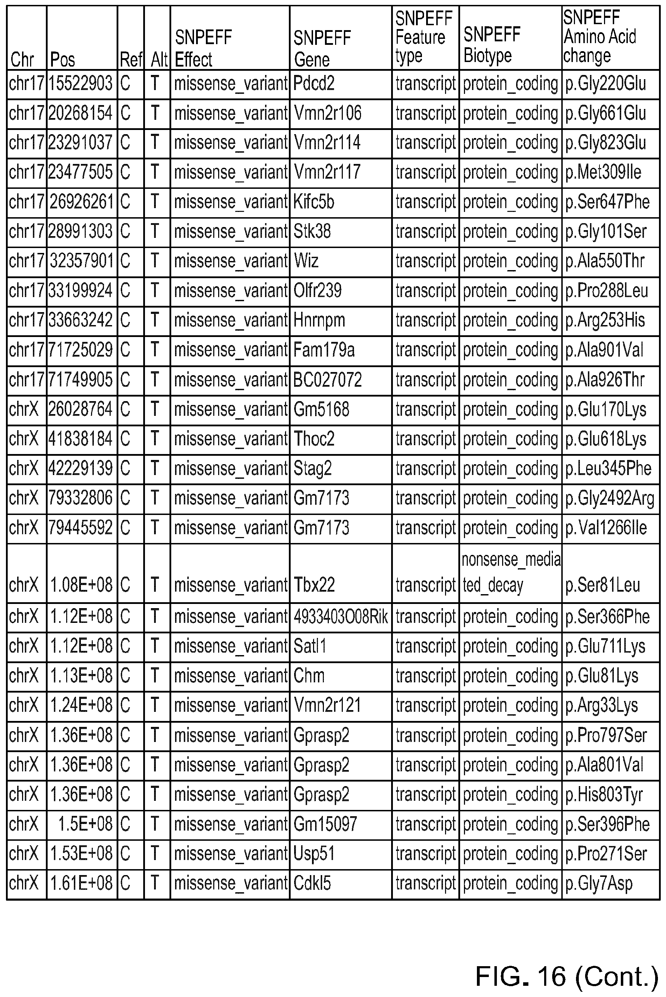

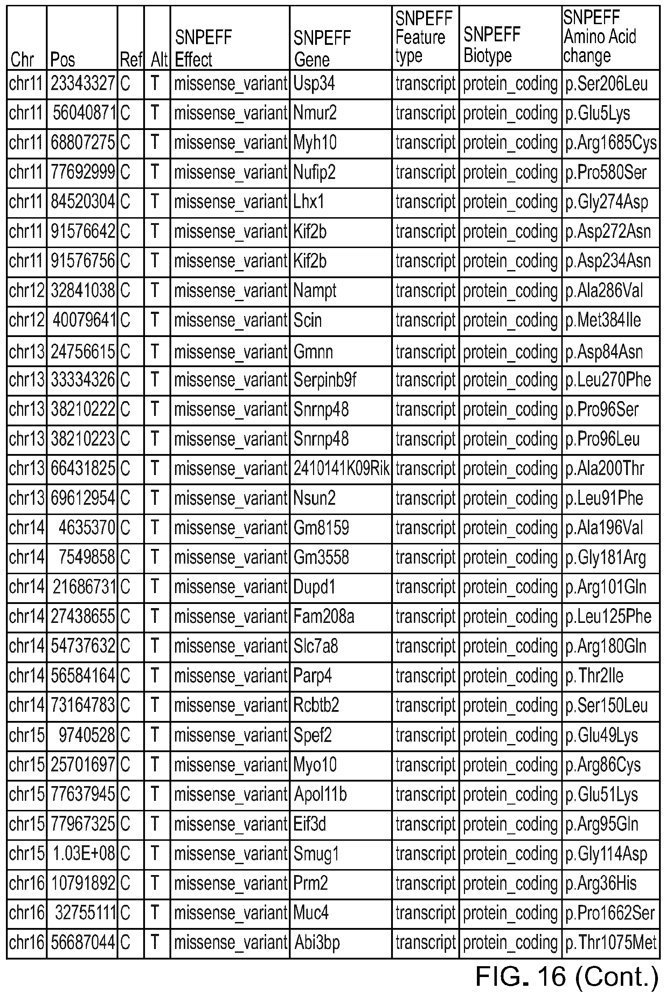

Figure 16 (Table 1). Amino acid changes induced by APOBEC3B. Mutations that resulted in a G to A or C to T transitions and an amino acid change.

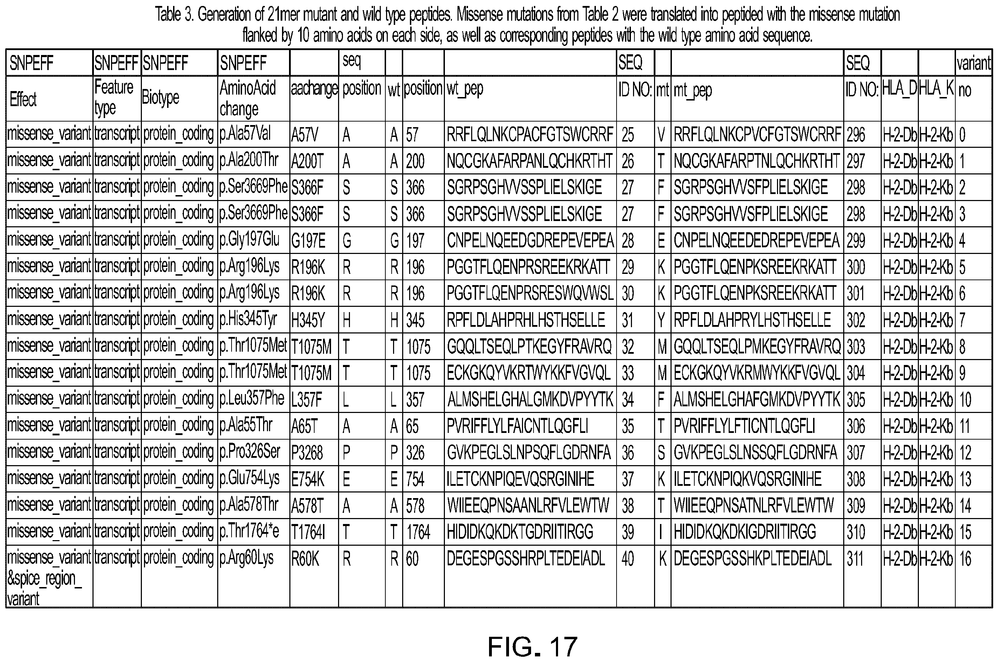

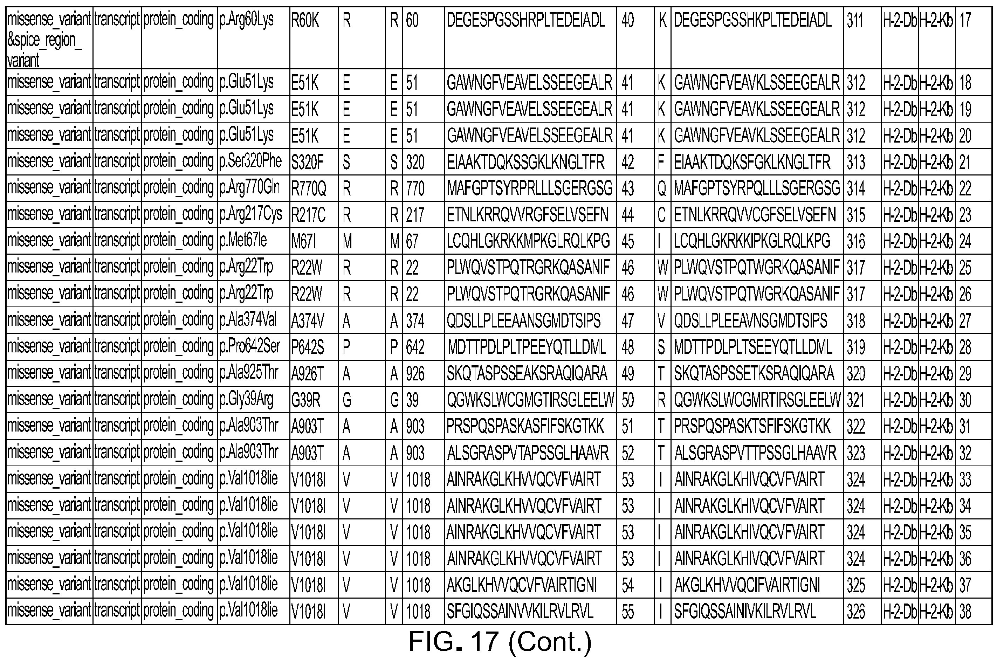

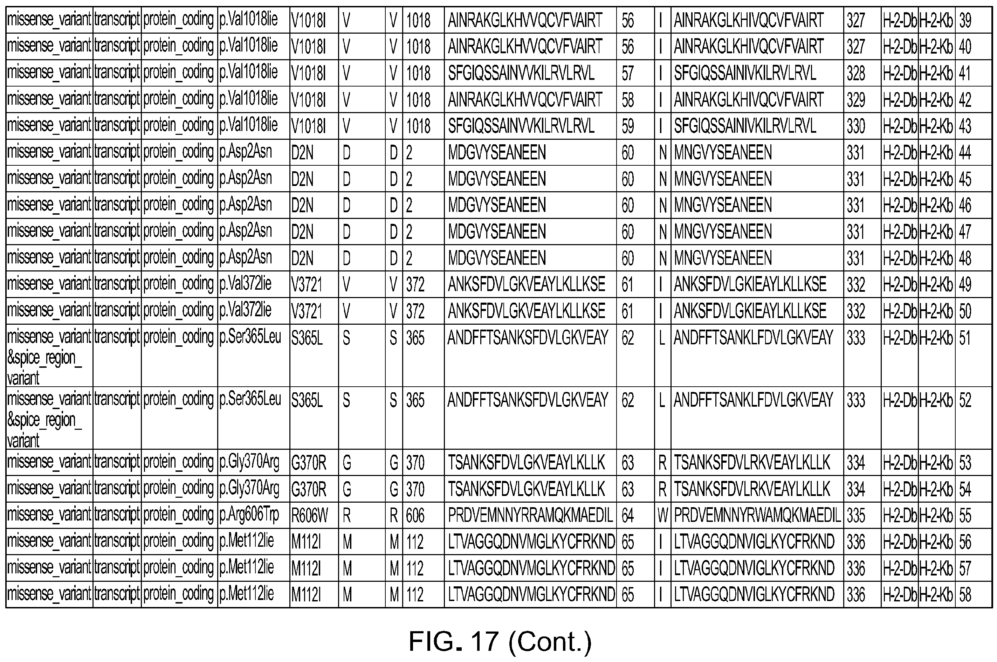

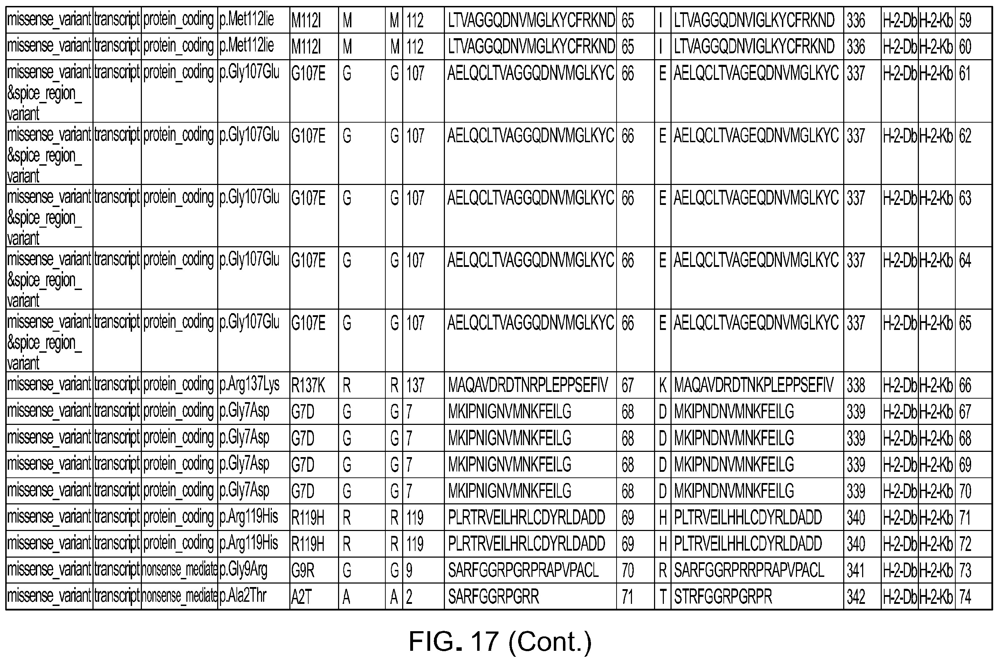

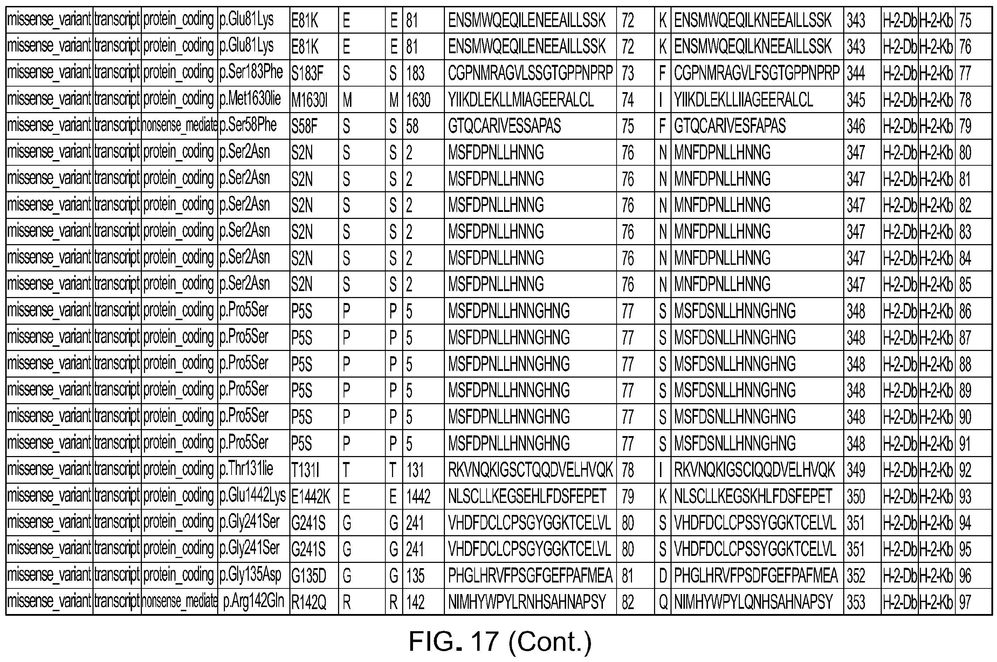

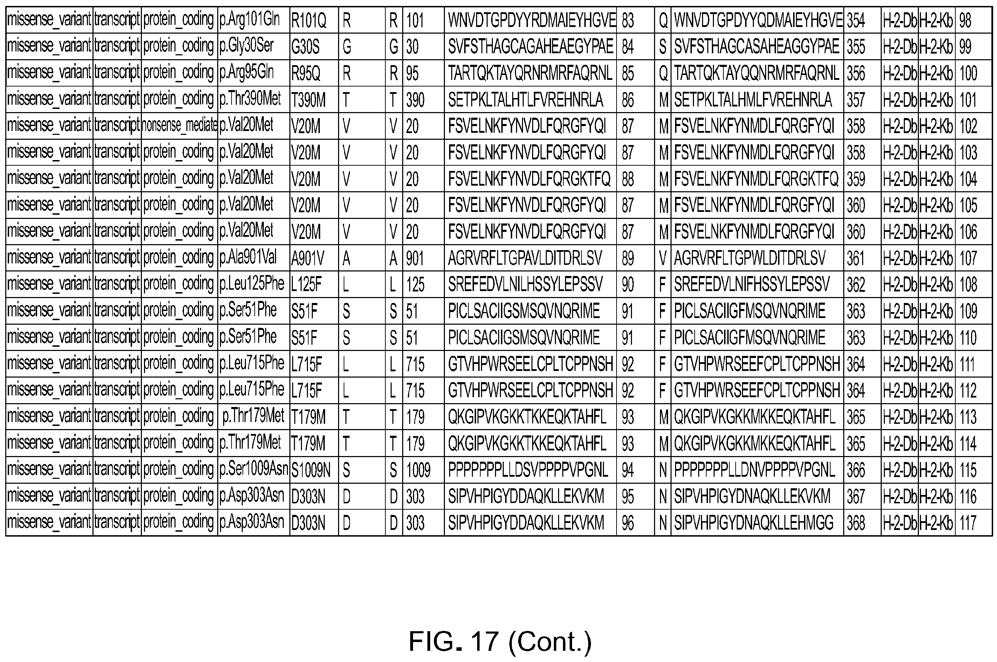

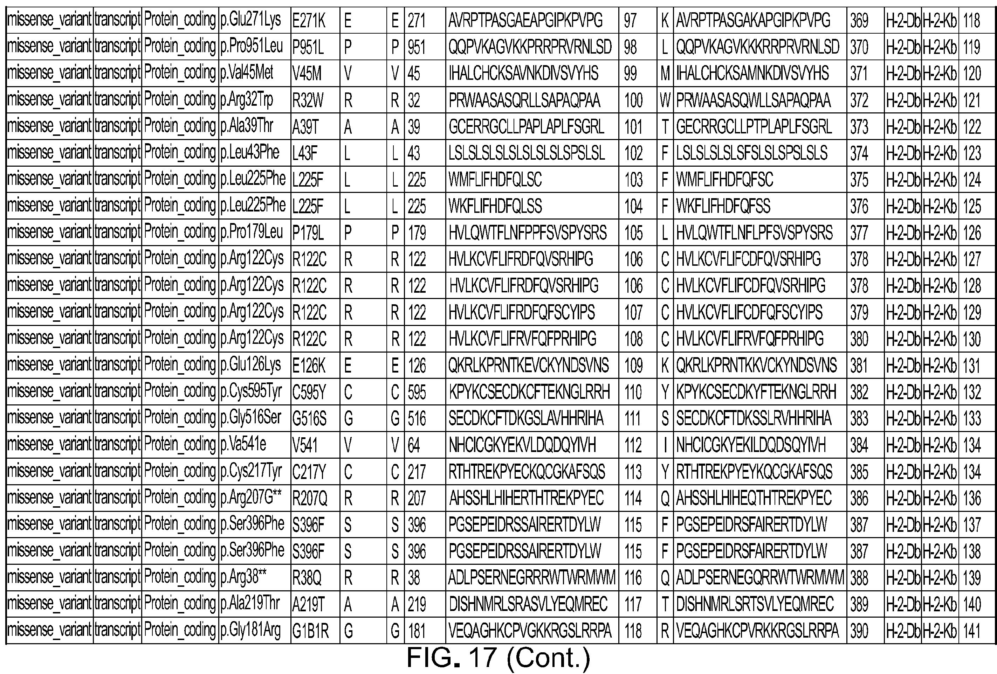

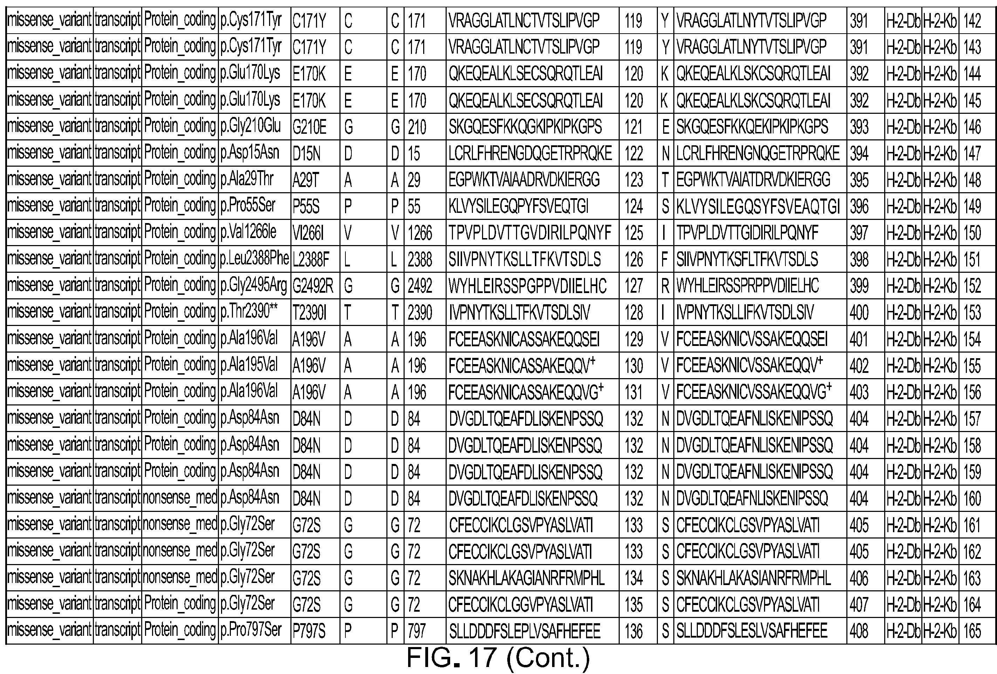

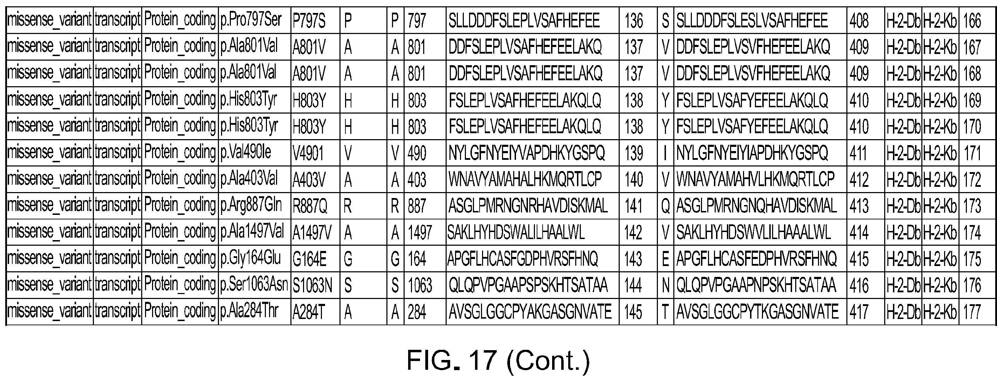

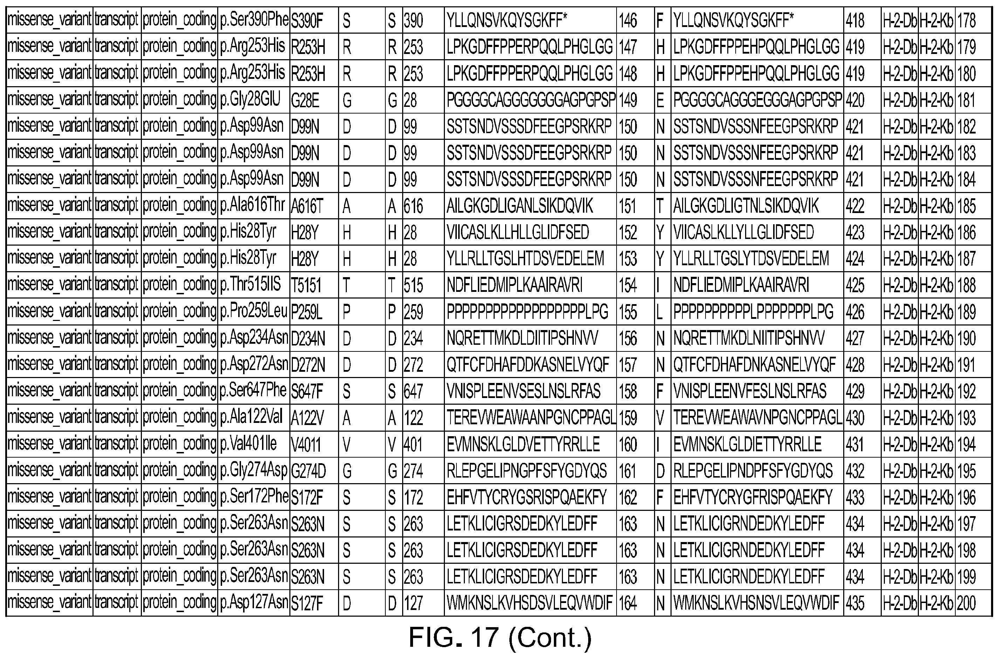

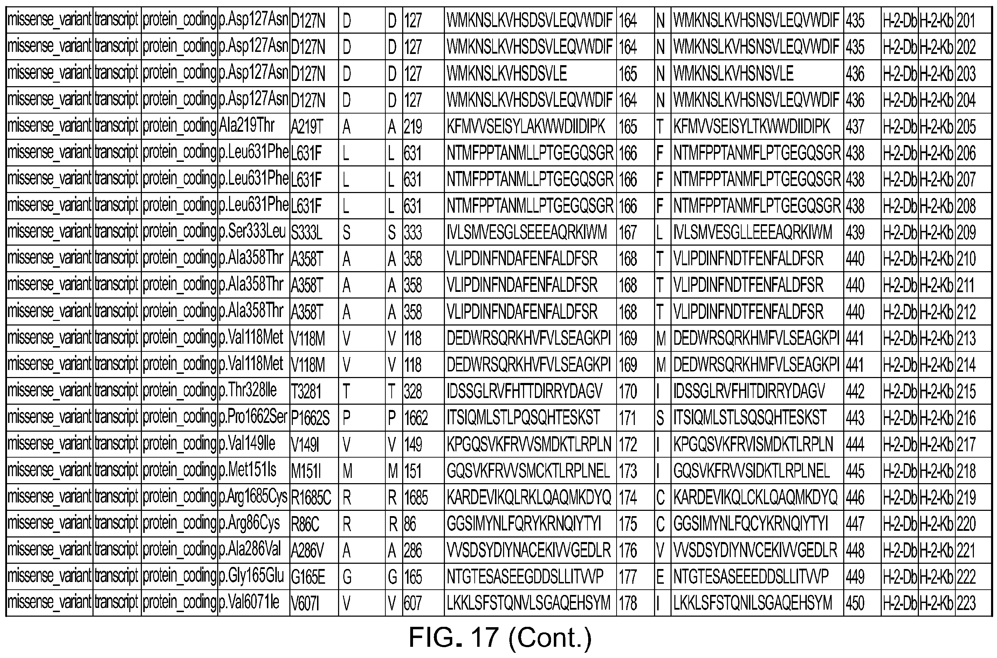

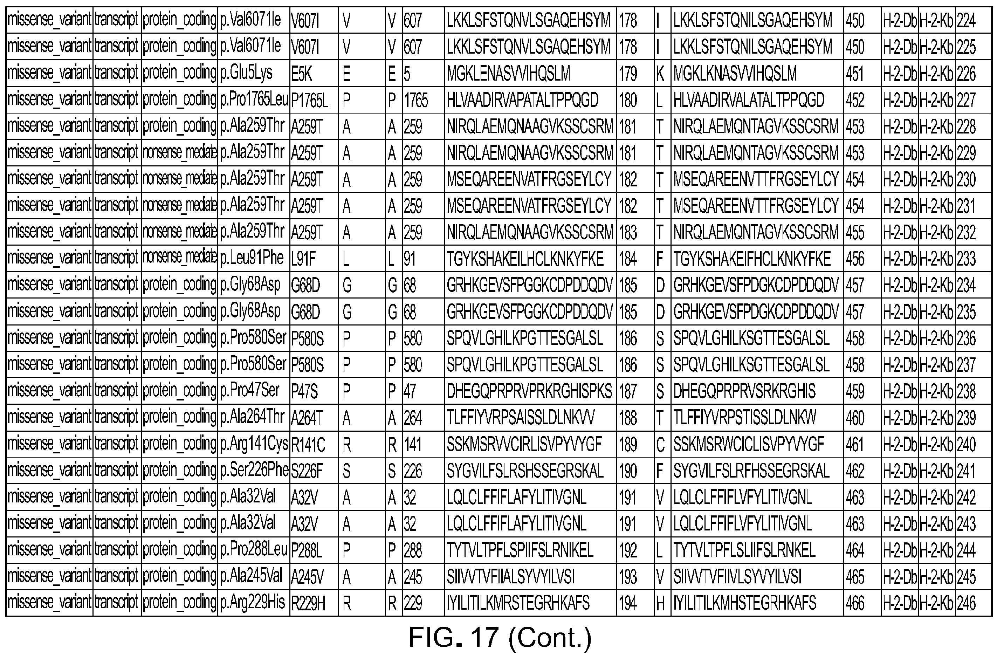

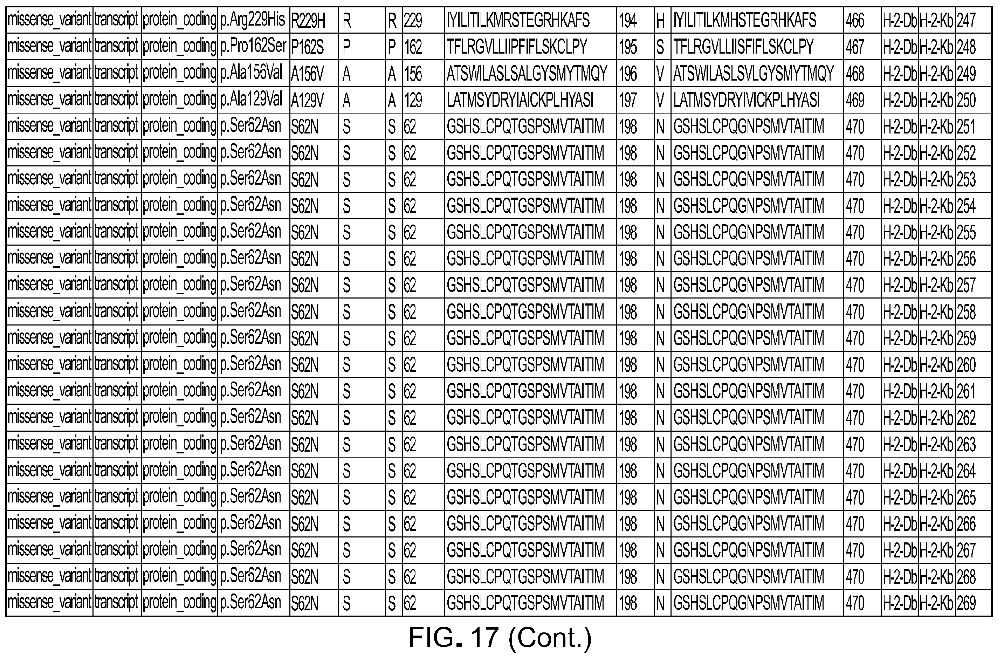

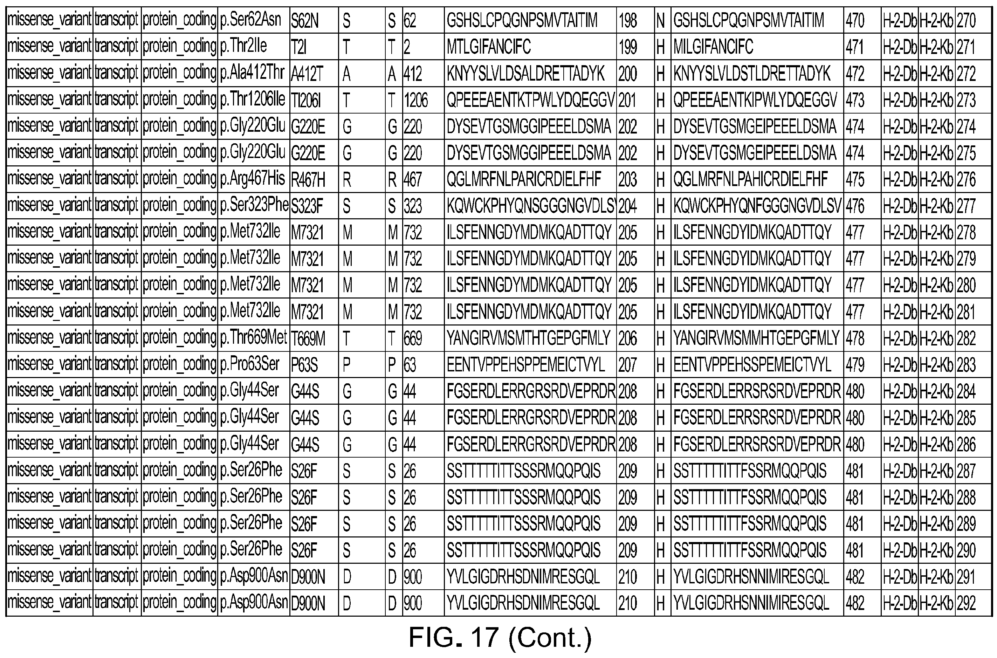

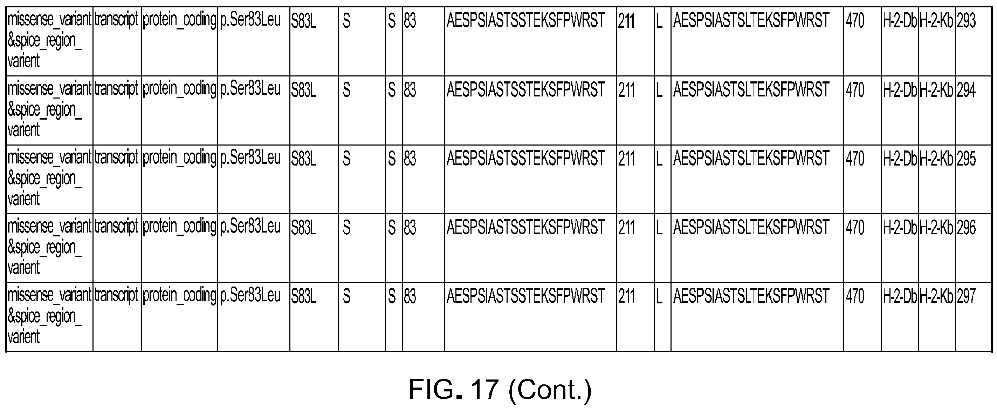

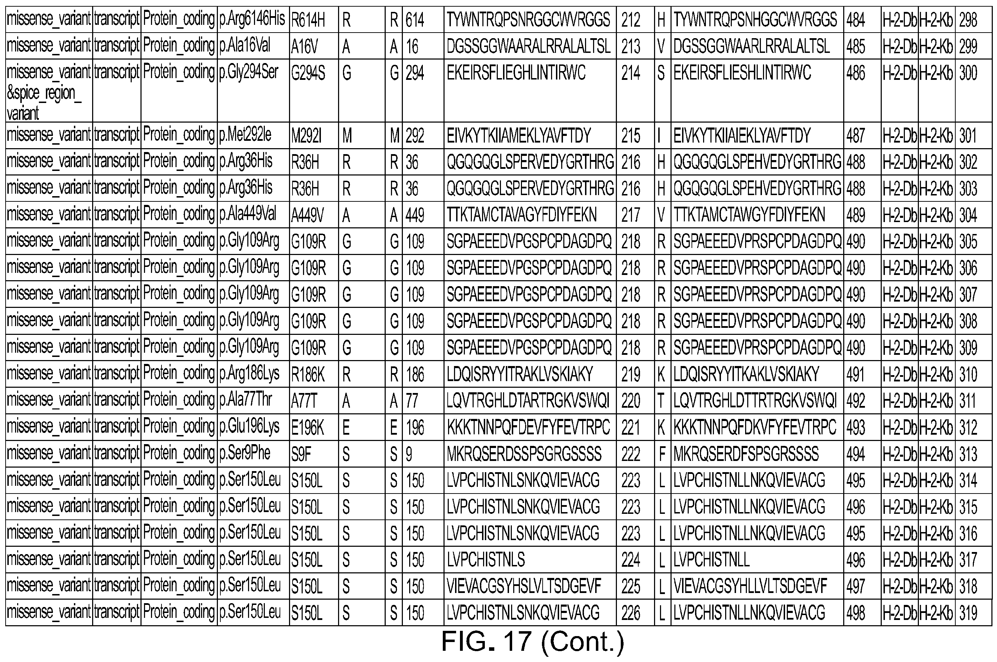

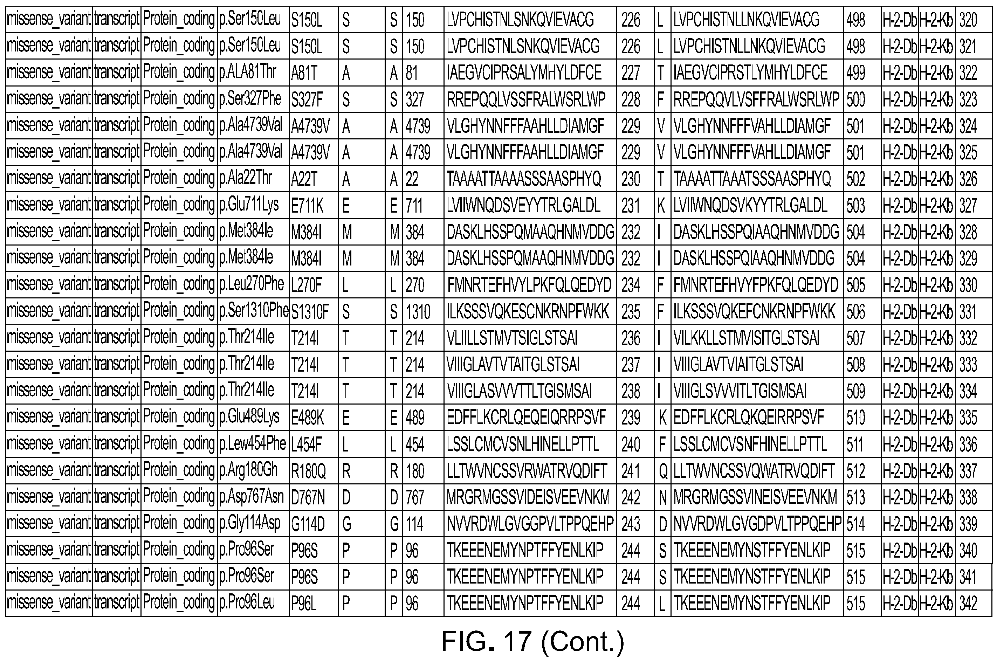

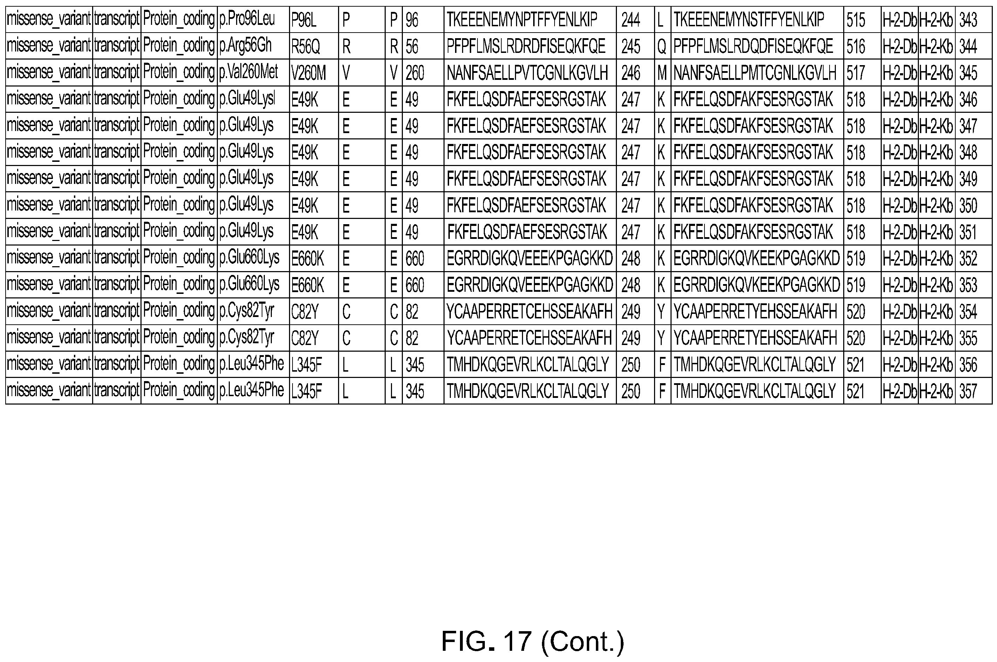

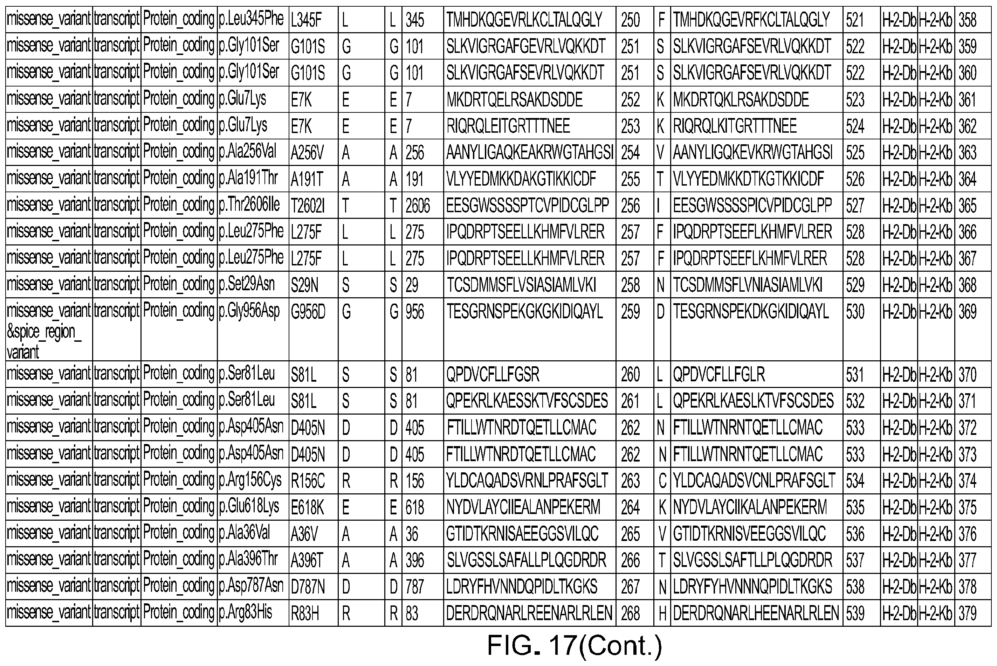

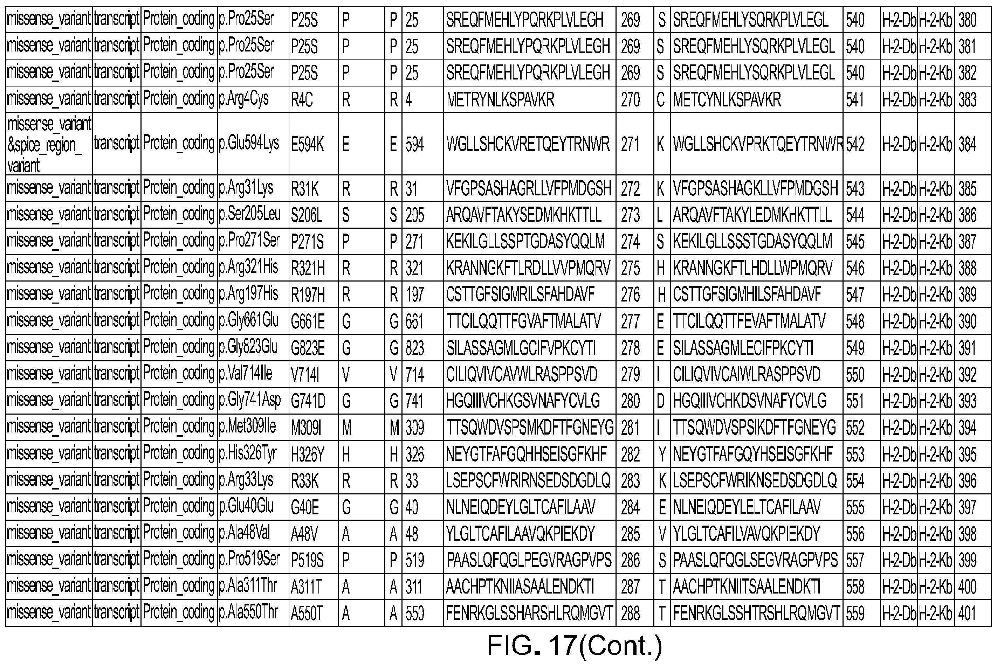

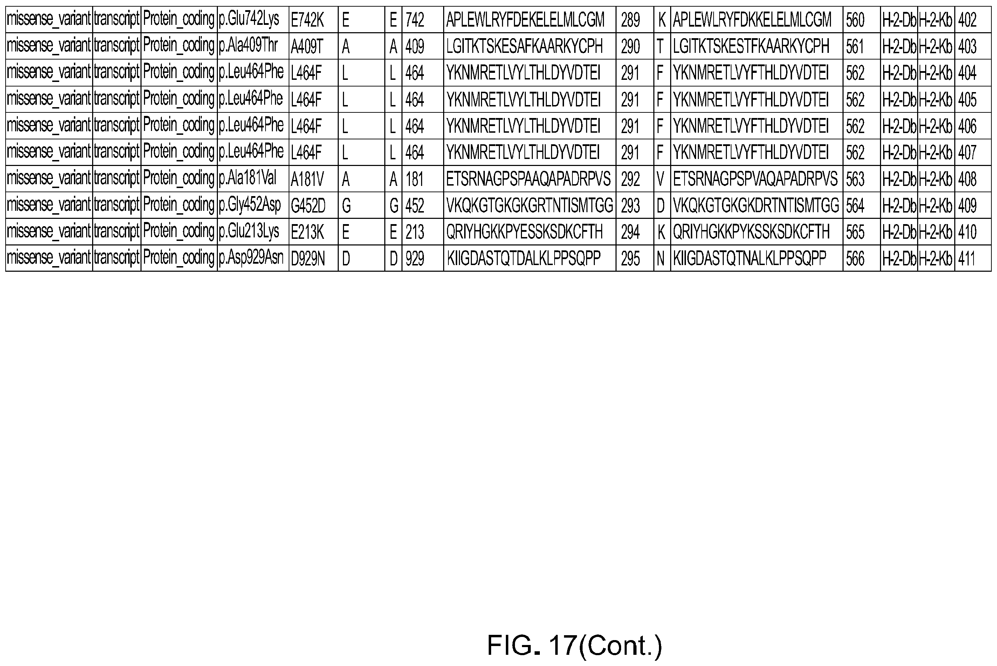

Figure 17 (Table 2). Generation of 21mer mutant and wild type peptides. Missense mutations from Table 1 were translated into peptides with the missense mutation flanked by 10 amino acids on each side, as well as corresponding peptides with the wild type amino acid sequence. Peptides shown include SEQ ID NOs: 25-566.

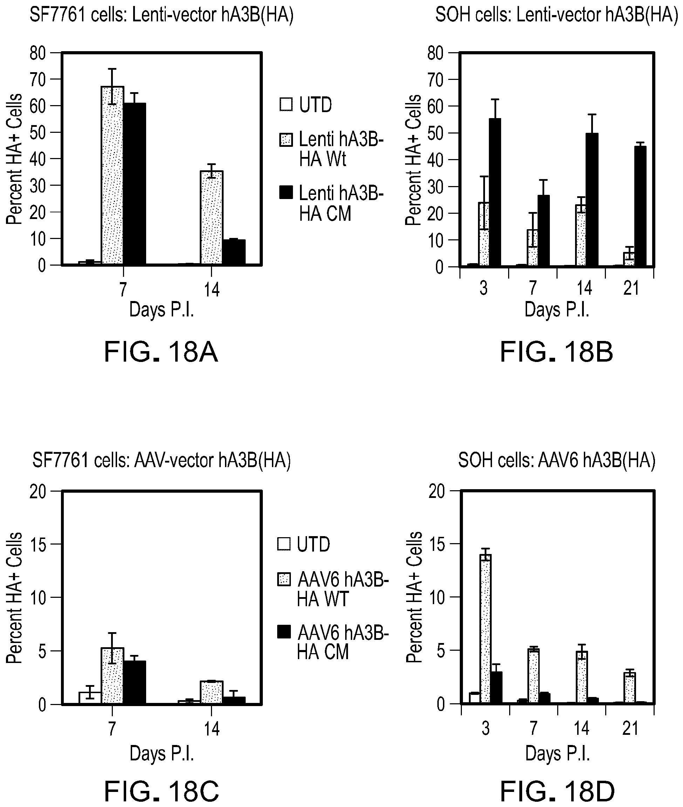

Figure 18. Protein expression of APOBEC3B from lentivirus and AAV vectors. (A,C) SF7761 DIPG cells were transduced with a lentivirus (MOI 3) or an AAV6 (5e3 genome copies/cell) vector, respectively, expressing the HA-tagged WT or catalytically inactive mutant human APOBEC3B. (B,D) SOH DIPG cells were transduced with a lentivirus (MOI 1) or an AAV6 (1e4 genome copies/cell) vector, respectively, expressing the HA-tagged WT or catalytically inactive mutant human APOBEC3B. Cells were harvested at the indicated timepoints and HA expression analyzed by flow cytometry. N=3/sample.

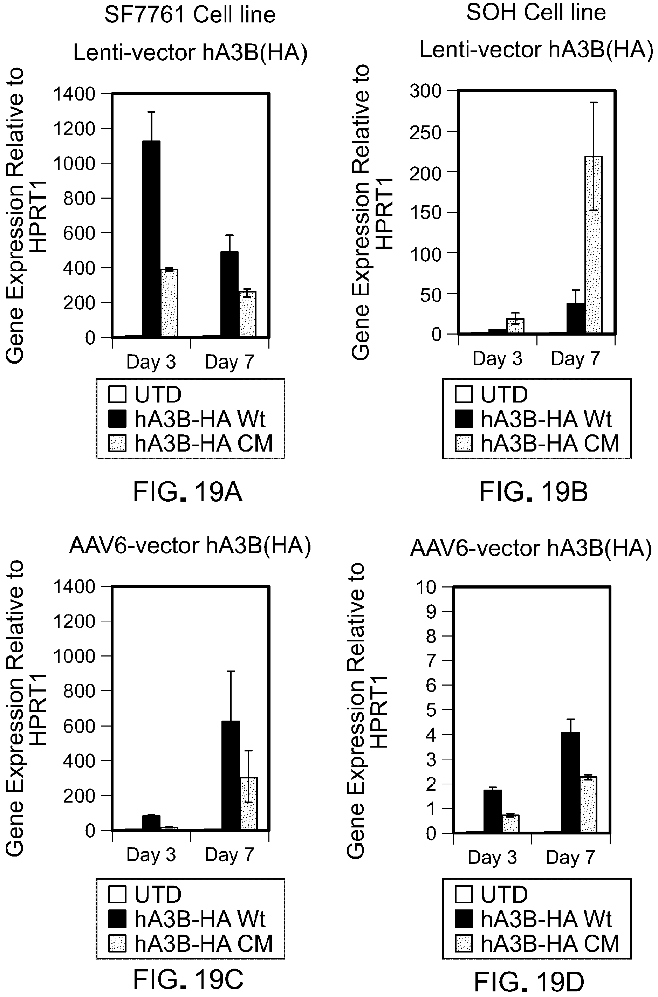

Figure 19. mRNA expression of APOBEC3B from lentivirus and AAV vectors. (A,C) SF7761 DIPG cells were transduced with a lentivirus (MOI 3) or an AAV6 (5e3 genome copies/cell) vector, respectively, expressing the HA-tagged WT or catalytically inactive mutant human APOBEC3B. (B,D) SOH DIPG cells were transduced with a lentivirus (MOI 1) or an AAV6 (1e4 genome copies/cell) vector, respectively, expressing the HA-tagged WT or catalytically inactive mutant human APOBEC3B. RNA from cells was harvested at the indicated time points, and qRT PCR was performed to determine the expression of APOBEC3B relative to the housekeeping gene HPRT1. N=3/sample.

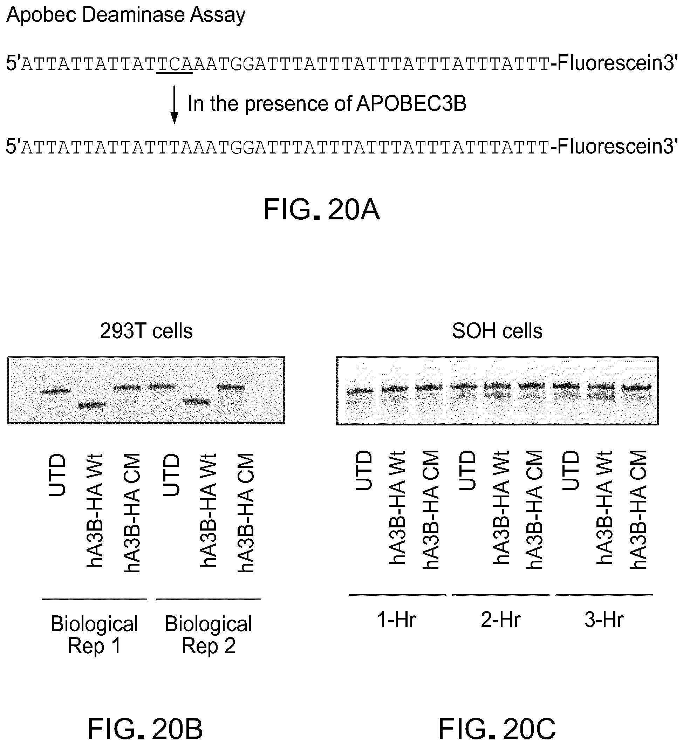

Figure 20. The APOBEC3B expressed from the lentiviral vector has catalytic function. (A) Assay principle. A fluorescent probe (SEQ ID NO:567) containing a single APOBEC3B target site (underlined) will be mutated in the presence of APOBEC3B to a mutated fluorescent probe (SEQ ID NO:568). This mutated site can be modified by UDG and cleaved by alkali treatment. Smaller cleavage products thus indicate the presence of APOBEC3B activity (see, e.g., Akre et al., PLoS ONE 11(5): e0155391 (2016)). (B) 293T transduced with a lentiviral vector expressing the WT or CM (catalytic mutant) human APOBEC3B. Cells were selected for 7 days in puromycin and lysed in HED buffer. Lysates were incubated for 1 hour at 37°C with in the presence of UDG with the 43-bp 3' FITC probe. Reactions were treated with 2M NAOH, heated at 95°C for 10 minutes and run on a 15% TBE urea gel and imaged it with a Typhoon imager. (C) SOH cells were transduced at an MOI of 2 with Lentivirus expressing the WT or CM (catalytic mutant) human APOBEC3B. 3 days post transduction, lysates were incubated with 3' FITC substrate described in panel A for the indicated periods of time.

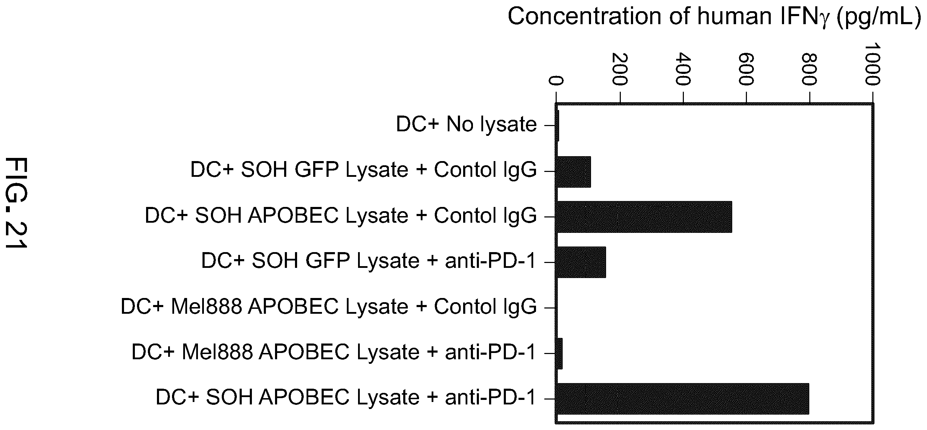

Figure 21. *In vitro* priming and recall response of human T cells with DC loaded APOBEC modified cell lysate. CD14+ monocytes and CD3+ T cells were harvested from the PBMCs of a healthy donor. Monocytes were differentiated into dendritic cells using GMCSF and IL4 and matured with TNFa, IL1b, IL6 and PGE2. Mature DCs were loaded with lysates of SOH cells transduced with lentivirus expressing GFP or APOBEC3B (equivalent of 10⁵ cells per 10⁴ DC). DC pulsed targets were cocultured with T cells at E:T of 10:1 for 5 days. CD3 cells were then purified, and co-cultured with fresh autologous DC loaded with SOH lysate for 72 hours. Interferon gamma (IFNg) was measured in the supernatant.

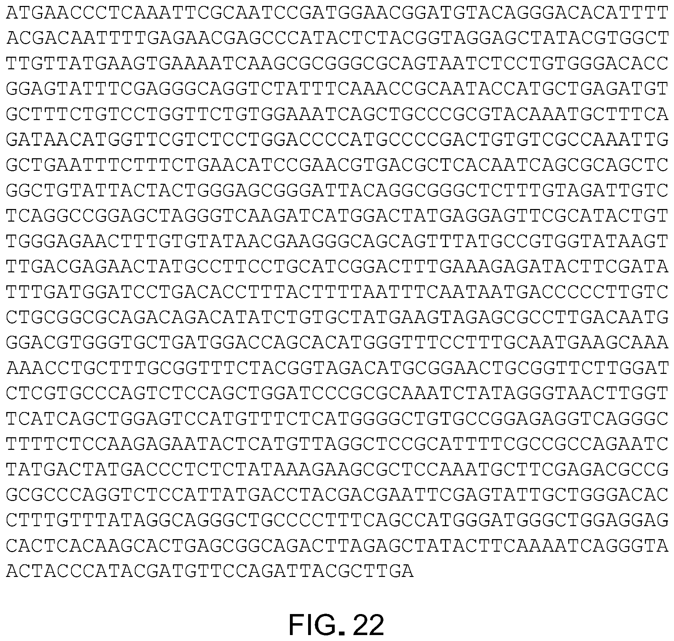

Figure 22. An exemplary nucleic acid expressing a wild type, human APOBEC3B polypeptide (SEQ ID NO:571).

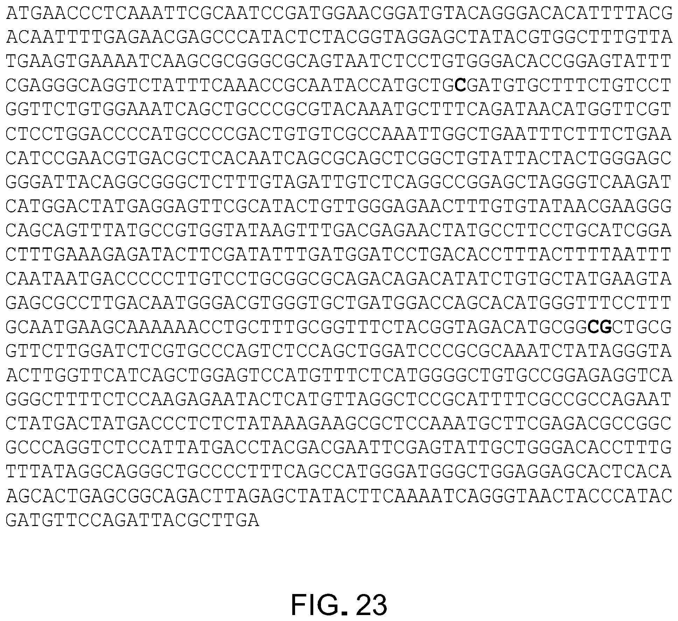

Figure 23. An exemplary nucleic acid expressing a catalytically inactive APOBEC3B polypeptide (SEQ ID NO:572). Nucleotides that are modified relative a nucleic acid expressing a wild type, human APOBEC3B polypeptide are shown in **bold** font.

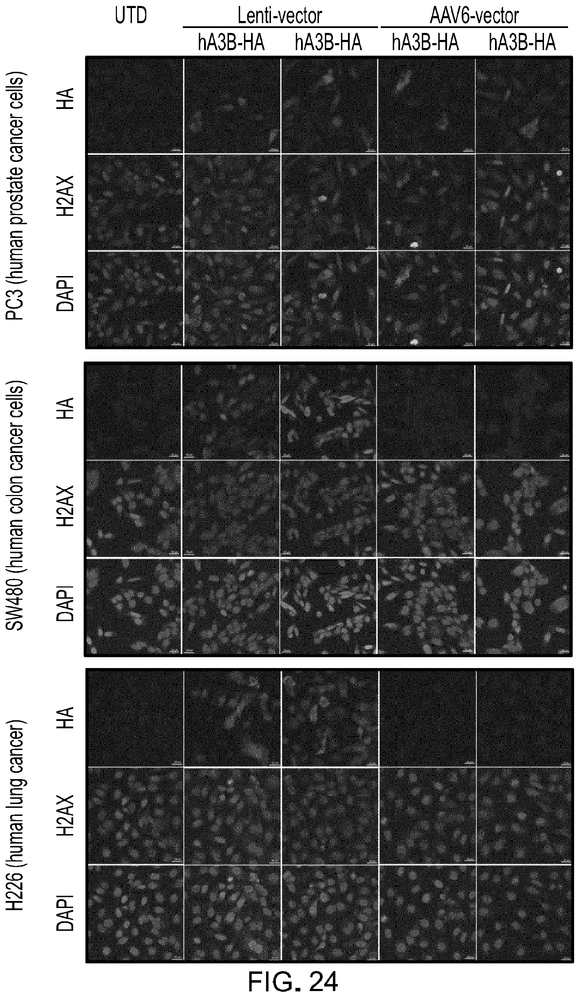

Figure 24. Representative fluorescent confocal microscopy images of three human cancer cell lines (PC3, SW480, H226) transduced with a lentiviral vector (MOI 1) or AAV6 (1e4 gc/cell) expressing the HA-tagged WT or catalytically inactive mutant human APOBEC3B. 48 hours post transduction, cells were reseeded in chamber slides and 24 hours later fixed and permeabilized. Cells were stained with rat-anti HA and rabbit anti-H2AX. Nuclei were counterstained with DAPI.

### DETAILED DESCRIPTION

This document provides materials related to treating a mammal having cancer. For example, this document provides compositions (*e.g*., vaccines) for treating cancer. The term "vaccine" as used herein refers to immunogenic compositions that are administered to a mammal for the prevention, amelioration, or treatment of diseases (*e.g*., cancer). In some cases, compositions provided herein can contain cells *(e.g.,* tumor cells) expressing APOBEC3 which can generate antigens *(e.g.,* neoantigens) having epitopes *(e.g.,* mutated epitopes) which are immunogenic in a mammal having cancer. This document also provides compositions for use in methods for treating a mammal having cancer. For example, the compositions *(e.g.,* vaccines) provided herein can be administered to a mammal (*e.g*., a human) having cancer to induce an immune response (*e.g*., an anti-tumor immune response) within the mammal. An anti-tumor immune response can be effective to reduce the severity of the cancer, to reduce a symptom of the cancer, and/or to reduce the number of cancer cells present within the mammal. A vaccine administered to a mammal can induce a protective immune response and/or a therapeutic immune response.

Expression of APOBEC3 in a cell *(e.g.,* a tumor cell) can induce mutations (*e.g.,* somatic mutations) which generate a library of antigens (*e.g*., tumor antigens such as mutated tumor antigens) reflecting a transcriptional profile of the cell (*e.g*., self-antigens, self-peptides, and self-epitopes). For example, a tumor cell expressing APOBEC3B can include a library of mutated tumor-specific antigens. In some cases, the library of antigens can include altered self-antigens, altered self-peptides, and/or altered self-epitopes.

Expression of APOBEC3 in a cell *(e.g.,* a tumor cell) can generate any appropriate antigens. In some cases, an antigen can be a tumor antigen *(e.g.,* a mutated tumor antigen). In some cases, an antigen can be a heteroclitic antigen. In some cases, an antigen can be a neoantigen. In some cases, an antigen can include one or more mutations (*e.g*., pre-existing mutations and/or non-pre-existing mutations). For example, when an antigen includes a mutation, the mutation can be a heteroclitic mutation. In some cases, an antigen can be a self-antigen. In some cases, an antigen can be a non-self antigen *(e.g.,* a xenogenic or foreign antigen such as a viral peptide).

Any appropriate cell can be used in compositions (*e.g*., vaccines) described herein *(e.g.,* containing one or more cells *(e.g.,* tumor cells) expressing APOBEC3). In some cases, a cell can be a tumor cell. In some cases, a cell expressing APOBEC3 can be an autologous cell *(e.g.,* from the mammal to be treated as described herein). In some cases, a cell expressing APOBEC3 can be an allogeneic cell *(e.g.,* from a mammal other than the mammal to be treated, such as cells from a donor mammal or a cells from a mammalian cell line).

Compositions *(e.g.,* vaccines) described herein *(e.g.,* containing one or more cells *(e.g.,* tumor cells) expressing APOBEC3) can include one or more cells expressing any appropriate APOBEC3. In some cases, the APOBEC3 polypeptide can be from any appropriate source. For example, the APOBEC3 can be a human APOBEC3 (*e.g*., any one APOBEC3A though APOBEC3BH, or any combination thereof). For example, the APOBEC3 can be a murine APOBEC3. For example, the APOBEC3 polypeptide can be a recombinant (*e.g*., chimeric) APOBEC3 polypeptide. In some cases, an APOBEC3 polypeptide can be an APOBEC3B polypeptide.

In some cases, compositions *(e.g.,* vaccines) described herein *(e.g.,* containing one or more cells *(e.g.,* tumor cells) expressing APOBEC3) can include one or more cells modified to express APOBEC3 polypeptides. APOBEC3 can be expressed using any appropriate method. In some cases, a nucleic acid expressing APOBEC3 can be introduced into a cell *(e.g.,* a cell to be used in a composition described herein). A nucleic acid expressing APOBEC3 can be any appropriate type of nucleic acid *(e.g.,* DNA, RNA, or a combination thereof). In some cases, nucleic acid expressing an APOBEC3B polypeptide can include the nucleic acid sequence set forth in SEQ ID NO:571 (Figure 22). Examples of nucleic acids that can be used to express APOBEC3 polypeptides include, without limitation, expression plasmids and viral vectors (*e.g*., adenoviral, AAV, lentiviral such as HIV-based lentiviral, or retroviral vectors). Nucleic acids that can be used to express APOBEC3 polypeptides can be introduced into a cell using any appropriate method. Examples of methods that can be used to introduce nucleic acids into cells include, without limitation, transfection and electroporation.

Compositions *(e.g.,* vaccines) described herein *(e.g.,* containing one or more cells *(e.g.,* tumor cells) expressing APOBEC3) can be administered to a mammal using any appropriate method. In some cases, compositions described herein can be administered to a mammal in the absence of any delivery vehicle. In some cases, compositions described herein can be administered to a mammal using a delivery vehicle such as one or more antigen-presenting cells (*e.g*., dendritic cells, macrophages, and B cells). For example, one or more cells expressing APOBEC3 polypeptides can be loaded into dendritic cells, and the dendritic cells containing the cells expressing APOBEC3 polypeptides can be administered to a mammal.

Any appropriate mammal having cancer can be treated as described herein. For example, humans and other primates such as monkeys having cancer can be treated with compositions *(e.g.,* vaccines) described herein *(e.g.,* containing one or more cells *(e.g.,* tumor cells) expressing APOBEC3) induce an immune response (*e.g*., an anti-tumor immune response) within the mammal within the human or other primate. In some cases, dogs, cats, horses, cows, pigs, sheep, mice, and rats having cancer can be treated with a composition (*e.g*., vaccine) described herein.

When treating a mammal (*e.g*., a human) having a cancer as described herein, the cancer can be any appropriate cancer. In some cases, a cancer treated as described herein can have a low mutational burden. Examples of cancers that can be treated as described herein include, without limitation, glioma (*e.g*., pediatric high grade gliomas (pHGGs) including anaplastic astrocytoma, glioblastoma multiforme (GBM), and diffuse intrinsic pontine glioma (DIPG)), melanoma, prostate cancer, lung cancer (*e.g*., non-small cell lung cancer), and colon cancer.

Once identified as having a cancer (*e.g*., glioma), a mammal can be administered a composition *(e.g.,* a vaccine) described herein *(e.g.,* containing one or more cells *(e.g.,* tumor cells) expressing APOBEC3). In some cases, methods described herein can include identifying a mammal as having a cancer *(e.g.,* a glioma). Any appropriate method can be used to identify a mammal having cancer. For example, imaging techniques and biopsy techniques can be used to identify mammals (*e.g*., humans) having cancer.

In some cases, a composition *(e.g.,* a vaccine) described herein *(e.g.,* containing one or more cells *(e.g.,* tumor cells) expressing APOBEC3) can be administered to a mammal having a cancer as a combination therapy with one or more additional agents used to treat a cancer. The one or more additional agents used to treat a cancer can include any appropriate cancer treatments. In some cases, a cancer treatment can include surgery. In some cases, a cancer treatment can include radiation therapy. In some cases, a cancer treatment can include administration of a pharmacotherapy such as a chemotherapy, hormone therapy, targeted therapy, and/or cytotoxic therapy. Examples of cancer treatments include, without limitation, administration of one or more immune checkpoint inhibitors (*e.g*., anti-PD-1, anti-PD-L1, anti-CLTA4, and/or anti-Tim3 antibodies). For example, a mammal having cancer can be administered a vaccine containing one or more tumor cells expressing APOBEC3 and administered one or more immune checkpoint inhibitors. In cases where a mammal having cancer is treated with a vaccine containing one or more tumor cells expressing APOBEC3 and administered and is treated with one or more immune checkpoint inhibitors, the one or more immune checkpoint inhibitors can be administered at the same time or independently. For example, the vaccine containing one or more tumor cells expressing APOBEC3 can be administered first, and the one or more immune checkpoint inhibitors administered second, or vice versa.

The invention will be further described in the following examples, which do not limit the scope of the invention described in the claims.

### EXAMPLES

### Example 1: Identifying a new class of tumor antigens

The family of APOBEC3 cytosine deaminase enzymes catalyze cytosine to uracil deamination of single stranded DNA (ssDNA) to generate C to T transitions, and to a lesser frequency C to G transversion mutations.

Whether a freeze-thawed whole tumor cell vaccine prepared from B16 melanoma cells stably overexpressing human APOBEC3B, or a catalytically inactive mutant (APOBEC3B MUT), could treat established subcutaneous parental B16 tumors was evaluated. While the B16APOBEC3B vaccine could modestly slow tumor growth as a single agent, a synergistic therapeutic effect was observed when combined with PD-1 checkpoint blockade and led to cures in 75-100% of mice (Fig 1A, B). In contrast, the vaccine produced from B16 cells overexpressing the catalytically inactive APOBEC3B did not provide any tumor control even in combination with anti-PD1. These data confirmed that APOEBEC3B induced mutations were necessary over and above the endogenous neoepitope repertoire of parental B16 cells to prime an adaptive anti-tumor response. Both a CD4 and CD8 response was elicited by vaccination, as depletion of either cellular compartment abrogated therapy (Fig 1B). Moreover, T cells isolated from mice treated with the vaccine and anti-PD1 as in Fig 1A produced very high levels of interferon (IFN)-y when co-cultured with parental unmodified B16 melanoma cells, the magnitude of which correlated with the observed therapeutic activity (Fig 1C). Of particular importance, no evidence of vitiligo associated with the APOBEC3B vaccine was seen, despite the robust anti-tumor therapy.

Whole genome sequencing of B16 APOBEC3B and APOBEC3B MUT overexpressing cell lines revealed the presence of 301 C to T or G to A missense mutations which were unique to the APOBEC3B line. These coding variants specific to the APOBEC3B cell line were filtered through an *in silico* MHC binding affinity algorithm to identify octamer or nonamer peptides whose increase binding affinity for H2K^{b} or H2K^{d} was below a threshold of 500 nM. A list of 36 candidates was further refined using the EMBL-EBI Expression Atlas for expression in skin tissue to identify 10 high affinity APOBEC3B-induced heteroclitic peptides (Fig 2A). Expression constructs were generated to encode a 10 polyepitope string separated by Ala-Ala-Tyr (AAY) spacers between the epitopes (see, *e.g.,* Velders et al., J Immunol 166:5366-5373 (2001)) and tagged with an N-end degron motif (see, *e.g*., Varshavsky, Protein Sci 20, 1298-1345, doi:10.1002/pro.666 (2011)), and transfected into B16 melanoma cells. The ability of the B16 cells modified by APOBEC3B to prime T cells against these heteroclitic peptide candidates was experimentally validated *in vitro* (Fig 2B). A specific IFNy recall response was detected from the T cells of mice vaccinated with the APOBEC3B vaccine upon restimulation with either the APOBEC3B-modified polyepitope construct or the wild-type polyepitope construct. Although the IFNy recall response from APOBEC3B vaccinated mice to the wild type sequences was significantly lower than to the mutated sequences, it was nonetheless clearly detectable. In contrast, no IFNy was detected in response to the wild type or mutated constructs from APOBEC3B MUT vaccinated mice.

The B16 melanoma model was a useful proof of concept for the altered-self library strategy, and whether the approach could be extended to an orthotopic brainstem model of HGG was determined. The location of the tumor in the brainstem poses potential problems for therapeutic approaches for this disease, including the risk of toxicity associated with an inflammatory reaction in an anatomically important region. Nonetheless, GL261 cells stereotactically implanted into the brainstem using previously published coordinates (Caretti, Brain Pathol 21:441-451 (2011)) were significantly responsive to treatment with an APOBEC3B-modified GL261 vaccine in combination with anti-PD-1 checkpoint blockade (Fig 3A). Importantly, neither acute nor chronic symptoms that could be associated with the therapy, or that could be indicative of neurological autoimmunity, were observed prior to sacrifice due to tumor burden. The priming of a T cell response by the APOBEC3B-modified GL261 vaccine was confirmed *in vitro* following co-culture of splenocytes from mice treated as in Fig 3A with parental unmodified GL261 cells (Fig 3B). As seen in the B16 vaccination model, the APOBEC3B- modified vaccine primed a modest T cell response that was significantly amplified through anti-PD-1 blockade.

Cumulatively, these data show that, when overexpressed in tumor cells, APOBEC3B generates a library of heteroclitic sequences which primes both CD4 and CD8 T cells that have escaped central tolerance and which recognize newly mutated antigens from the vaccine. Critically, these T cells generated against the APOBEC3B-induced somatic mutations in the cell vaccine are also able to recognize the corresponding unaltered self epitopes expressed on the tumor cells (Summarized in Fig 4). There are three core principles that underlie the current proposal: (1) Dendritic cells are the most potent antigen presenting cell type and *ex vivo* differentiation, maturation, and loading of antigen, circumvents tumor microenvironmental limitations; (2) a paucity of targetable immunogenic neoepitopes in a tumor can be overcome through the introduction of mutations *ex vivo* which function as heteroclitic peptides in a vaccination setting; and (3) integration of immune checkpoint blockade (ICB) into the vaccination schedule amplifies the anti-tumor T cell response to provide a synergistic therapeutic effect.

### Example 2: APOBEC3B tumor cell vaccine platform in a genetically engineered mouse model of pHGG

GL261 tumors are a modestly representative model of HGG and can be implanted into the brainstem. However, they do not recapitulate the molecular features that are unique to pediatric HGGs. Antigenetically engineered mouse model (GEMM), which anatomically and molecularly captures the features of DIPG was used. The tumors arise from Nestin expressing neural progenitor cells which are thought to be the cell of origin of DIPGs (Becher et al., Cancer Res 70:2548-2557 (2010)), and progress in the context of the developing brain under the influence of immunoediting. A breeding colony was established for the Nestin tv-a/p53 floxed mice and validated that when DF-1 producer cells transfected with the RCAS plasmids are injected directly into the brainstem of neonatal mice, brainstem tumors develop within approximately 4-6 weeks post injection and necessitate euthanasia (Halvorson et al., PLoS One 10:e0118926 (2015)) (Fig 5A). It was confirmed histologically that the tumors are located in the pons or midbrain regions of the animals and that the tumor is invasive into the adjacent brain parenchyma (Fig 5B and C).

**APOBEC3B mutational burden induced through various vector or transfection platforms for therapeutic vaccination.** Using an explanted cell line from the RCAS DIPG model, and a retroviral pBABE vector encoding APOBEC3B, it was validated the APOBEC3B modified tumor vaccines can prime an adaptive T cell response to provide therapy in this genetically engineered mouse model in an analogous manner to the B16 and GL261 models. To translate this concept to a clinical approach, it was functionally defined how the duration and expression level of APOBEC3B correlates with (1) the frequency and reproducibility of the induced mutations in tumor lines, (2) the magnitude of the CD4 and CD8 T cell response, and (3) the overall therapeutic value of the vaccine in the GL261 orthotopic model and the RCAS DIPG GEMM.

Although the retroviral expression system has worked well, alternative methods to introduce APOBEC3B were explored that provide more transient expression and which may be more amenable to meet FDA requirements for product manufacture. To this end, an E1/E3 deleted replication incompetent Adenovirus was constructed using the ViraPower Adenoviral Expression System (ThermoFisher) that expresses APOBEC3B using an inducible Cre-LoxP system and has enabled us to overcome difficulties associated with overexpression of APOBEC3B during viral rescue. A pCDNA plasmid construct expressing APOBEC3B was used. Retrovirus or adenovirus infection or plasmid transfection is conducted at various multiplicities of infection (MOIs) or concentrations of DNA, and cellular RNA is isolated following multiple population doublings for RNA sequencing using the TruSeq Stranded mRNA Library Prep Kit (Illumina). The coding transcriptome was interrogated to simultaneously identify single nucleotide variants and short indels introduced by APOBEC3B, and to provide an abundance value for both APOBEC3B and putative neoepitope peptides, as the MHC immunopeptidome is enriched in peptides derived from highly abundant transcripts (Fortier et al., J Exp Med 205: 595-610 (2008)). RNA-seq analysis is done in collaboration with the Biostatistics and Bioinformatics Core at Mayo Clinic. Briefly, reads from the parental explanted cell line, as well as those from the APOBEC3B-modified lines, is mapped to the mouse genome (GRCm38 / mm10) using the STAR aligner. RNA-seq identified variants is called using the Broad Institute's Unified Genotyper and Haplotype Caller from their Genome Analysis Tool Kit, and an expression value will be quantified for each gene from Subread's featureCounts software. Variants will be filtered and annotated using multiple tools including; Ensembl's Variant Effect Predictor, Harvard's SIFT/PolyPhen, and the Wellcome Trust Centre's Clinical Annotation of Variants algorithms to identify C to T missense mutations with an adjacent 5'T to identify likely APOBEC3B induced mutations. Finally, candidate 15-mer long peptides with 7 amino acids flanking the mutated amino acid on either side will be fed into the NetMHC4.0 algorithm (as in Fig 2) to predict their MHC class I H2D^{b} and H2K^{b} and MHC class II IA^{b} binding affinity.

For each snapshot time point at which RNA is isolated, and for each APOBEC3B expression method, a freeze thawed vaccine is prepared and administered to RCAS DIPG or GL261 tumor bearing mice in combination with PD-1 checkpoint blockade as described for the GL261 and B16 models. The impact on overall survival is assessed, and a subset of mice will be sacrificed for immune monitoring. T cells is harvested from spleens and cervical lymph nodes, labeled with CFSE, and co-cultured with the parental RCAS DIPG or GL261 cells *in vitro.* The proliferation of these T cells, as well as their polyfunctional cytokine response (IFNy, TNFα, IL2), is measured by intracellular staining and flow cytometry and used as a metric of the magnitude of the potency of the vaccine. These assays are performed with T cells restimulated with peptide pools containing the wild type sequence or the putative high affinity neoeiptopes predicted with NetMHC4.0. Thus, the overall mutation burden, the frequency of the mutations, and the abundance of transcripts which are predicted to contain APOBEC3B- induced mutations and to bind to MHC with high affinity, is correlated with the therapeutic value of the vaccine.

**Evaluation of potential toxicity associated with the priming of T cells recognizing heteroclitic epitopes specific to pHGG.** No evidence of vitiligo was observed in mice treated with the APOBEC3B-modified B16 tumor cell vaccine as a single agent or in combination with immune checkpoint blockade, nor was evidence of neurological autoimmune symptoms associated with the APOBEC3B-modified GL261 vaccine observed. Autoimmunity has neither been observed following vaccination with a VSV based altered self cDNA libraries. T cells that have escaped central tolerance and which weakly bind to self antigens, but can be primed and expanded through heteroclitic peptide vaccination, remain relatively weak as single clonal populations. Thus, the selectivity of the approach relies on the cumulative therapeutic strength of this polyclonal repertoire of T cells which recognize the neoplastic immunopeptidome signature presented on a tumor cell, but that differs from untransformed cells.

An evaluation of any potential toxicity that may be associated with the APOBEC3B induced altered-self-library vaccination approach is done. In order to monitor the potential pathological role that T cells primed with an APOBEC3B- modified vaccine may have, T cells are harvested from the cervical lymph nodes and spleens of tumor bearing mice that are vaccinated with APOBEC3B- or catalytically inactive APOBEC3B- modified tumor cells and adoptively transferred to non-tumor bearing recipient mice following a dose escalation ladder. Recipient mice are monitored for the development of neurological symptoms for at least 150 days, and serial snapshot histological analysis is performed to verify whether a T cell infiltrate and corresponding tissue destruction is present (Mayo Clinic Arizona Pathology Core). In order to push this system, adoptive T cell transfers and the above described readouts is performed for mice which receive a sham surgery to cause physical trauma and induce an inflammatory response which may additionally recruit T cells. Should autoimmunity be observed under these circumstances, it is evaluated whether current clinical protocols for the management of autoimmunity, such as the employment of corticosteroids for patients receiving immune checkpoint blockade, can mitigate the pathology.

**Integrate the APOBEC3B-modified whole cell vaccine into a clinically analogous dendritic cell vaccination platform and optimize adjuvant therapy.** Inactivated whole tumor cell vaccination is a relatively inefficient method of vaccination and the magnitude of the observed therapeutic response speaks to the increase in immunogenicity gained by APOBEC3B modification. This novel repertoire of heteroclitic peptide targets is leveraged by loading bone marrow derived dendritic cells (BMDCs) with tumor cells modified with APOBEC3B. Murine BMDCs are differentiated in GMCSF and matured with TNFα and PGE2. To mimic a clinical protocol for the generation of tumor cell lysate, APOBEC3B-modified cells are resuspended in a hypotonic solution and freeze thawed three times and fed to dendritic cells. Dendritic cells are administered to tumor bearing mice within 24 hours of antigen loading. The magnitude of the CD4 and CD8 T cell response are compared between mice receiving freeze thawed lysate vaccine and tumor cell loaded BMDCs by flow cytometry following restimulation of T cells *in vitro* with parental RCAS DIPG tumor cells or peptides identified. The overall therapeutic effect of the DC vaccine compared with freeze thawed lysate vaccine is compared in mice which additionally receive anti-PD1, anti-CTLA-4, anti-Tim3, or combinations of ICB, or control IgG. T cells from mice vaccinated in this manner is adoptively transferred to recipient sentinel mice to monitor the potential development of autoimmune toxicity, as described.

**Statistical Analyses.** For Aim 1, 10 mice per group are used for survival analysis. If censoring exists, survival across groups are compared using the log-rank test. If censoring does not exist, then a Student's t-statistic (2 groups) or analysis of variance (ANOVA) (>2 groups) will be utilized to compare survival times. For studies with >2 groups, the overall F-statistic will be evaluated first; if the F-statistic p-value <0.05, then contrast statements will be used to test for pairwise comparisons of interest. Importantly, the studies described in Aim 1 include multiple "control" groups that while they are important to verify that the animal study was performed correctly, are not of biological interest. For all analyses, α=0.05 will be used. Assuming α=30, 10 mice per group will provide 80% power to detect a difference of 40 days, with a two-sided α=0.05. Alternatively, assuming σ=6 a difference of 10 days can be detected. As demonstrated in Fig 1 and 3, large effect sizes are expected and statisticallysignificant changes with 7-8/group can be detected, highlighting the feasibility of these studies. For *in vitro* analysis of T cell responses, 4-5 mice per group are compared using ANOVA models.

### Example 3: Transduction protocol for the preparation of APOBEC3B mutation induced tumor cell vaccines using a biobank of primary human pHGG cell lines

The preclinical development and testing of novel therapeutic approaches for pHGG and DIPG in particular has been limited by the scarcity of human cell lines that authentically represent the disease. Although surgical resection for DIPG tumors is not possible, biopsies are routinely performed at Mayo either with a stereotactic needle or in an open fashion depending on the tumor characteristics for each patient. This practice has enabled the procurement of sufficient tissue to generate a novel biobank over the last three years of cell lines for *in vitro* and xenograft testing. To date, the pediatric brain tumor biobank includes 20 novel cell lines which capture a variety of histological subtypes, including DIPG, medulloblastoma, ependymoma, meningioma, GBM, and others. The presence of mutations common to DIPG within novel cell lines and in the context of cell lines obtained from collaborators at other institutes (Fig 6A) was evaluated. In addition to this molecular characterization, RNA-seq was performed on this subset of DIPG lines (Fig 6B).

**APOBEC3B mutational burden in human DIPG cell culture conditions.** Having established a target range of APOBEC3B- induced mutations that optimally primes a T cell response that can cross react and provide therapy against a parental tumor, a protocol was developed to achieve this same mutational burden in human cell lines using human-specific cell culture reagents. B16 and GL261 cells are highly cell culture adapted and due to their rapid rate of division APOBEC3B overexpression is only tolerated for up to three weeks before the cells either transcriptionally silence the transgene or die as a result of the accumulation of mutations in key genomic loci. There is both heterogeneity in the growth characteristics within the primary cell bank of cell lines which grow as tumor neurospheres and as adherent lines, and substantial differences from the murine B16, GL261 lines, and from the explanted RCAS DIPG line. Using three DIPG lines (MC PED-17, SU-DIPG-IV, and SU-DIPG-VI) which represent distinct H3.3 and H3.1 H27M mutation classes, and transcriptional profiles (Fig 6), APOBEC3B was introduce using the adenoviral or retroviral vectors, or through plasmid transfection, and perform an analogous profiling of APOBEC3B-induced mutations by RNA-seq as described after multiple population doublings. Together the data correlating the growth rate of the cells, method of culture, the method of APOBEC3B introduction, and the overall induced mutational load, allows us to develop an algorithm to consistently induce the target mutational burden in novel cell lines.

APOBEC3B modified tumor vaccination strategy in an *in vitro* priming assay using healthy human donor peripheral blood cells. The timescale required for therapy in rapidly progressing pHGG patients is not compatible with the generation of sufficient autologous tumor material to prepare a DC vaccine and therefore necessitates a pre-existing allogeneic tumor cell bank for off the shelf use. Studies described (see, *e.g.,* Fig 1-3) have been, or are, performed in genetically identical mice. As the allogeneic setting already presents a degree of altered self, how the target mutation burden identified overlays on a pre-existing set of non-tolerized neoepitopes is explored. Using an established *in vitro* T cell priming assay (Prestwich et al., Clin Cancer Res 15:4374-4381 (2009)), the ability of DCs presenting lysate from APOBEC3B- modified human DIPG cell lines bearing variable mutational burdens to prime a polyclonal T cell response which can cross react back on the parental unmodified cell immunopeptidome is evaluated.

Peripheral blood mononuclear cells (PBMCs) are collected from healthy donors, and microbead sorted CD14⁺ monocytes will be differentiated into DCs in the presence of GMCSF and IL4, and matured with TNFα and PGE2 in serum free X-VIVO media, according to the established DC workflow. Monocyte derived DCs (moDCs) are loaded with APOBEC3B-modified DIPG cell lysate and co-cultured with autologous healthy T cells for two weeks in the presence of additional IL2 and IL7. *In vitro* primed T cells, as well as uncultured T cells, are labeled with CFSE and restimulated with moDCs loaded with lysate from either the parental unmodified pHGG cell line or the APOBEC3B- modified line. The proliferation of the T cells and their ability to produce IFNy, TNFα, IL2 is measured by flow cytometry. The cytokine recall response is evaluated in the presence or absence of checkpoint blocking antibodies. The ability of fluorescently labeled moDCs loaded with no cell lysate, unmodified DIPG cell lysate, or APOBEC3B- modified cell lysate, to act as targets and be specifically killed by *in vitro* primed T cells in a VITAL assay (Hermans et al., J Immunol Methods 285:25-40 (2004)) is evaluated.

A second important aspect of vaccination with allogeneic tumor material is the conservation between the peptidome of the cell line lysate and the recipient patient tumor. The relative abundance of the peptides presented by the DCs can shape the priming of the novel tumor-specific T cell repertoire. The *in vitro* T cell priming assay is used to evaluate the importance of conservation of expression patterns on the T cell recall response to DCs loaded with tumor lysates with varying degrees of dissimilarity to the original priming lysate. The RNA-seq determined hierarchical clustering (Fig 6B) is used to evaluate recall with dissimilar histone mutations, as well as more histologically unrelated tumor types from the biobank such as a GBM or medulloblastoma. In parallel, the DIPG RCAS and GL261 mouse models are used to investigate the magnitude of the recall response of T cells from DIPG vaccinated mice to GL261 and vice versa.

### Example 4: APOBEC3B pHGG cell banks for use in clinical trials for pediatric high grade glioma

The workflows established for the pHGG biobank and the adult GBM GMP cell bank used in the previous DC vaccine trial (MC1272; NCT01957956) are expanded. Biopsy or surgical specimens are pathologically confirmed and expanded using a clinical scale Good Manufacturing Practice (GMP) compliant production protocol in a proprietary culture medium that exploits the growth factors and cytokines found in human platelets that promotes *in vitro* growth of primary tumors. Platelets are cell bodies released from megakaryocytes and that express growth factors such as EGF, FGF, VEGF, and PDGF. The generation of the novel APOBEC3B modified GMP lines proceeds in two distinct steps: (1) the establishment, expansion, and characterization of the novel cell lines; and (2) the introduction of APOBEC3B through the gene delivery methods described herein. The expression of a variety of markers to evaluate their differentiation state (Nestin, GFAP, olig2, β-tubulin) is evaluated and the presence of additional tumor associated antigens that are commonly overexpressed in pHGG (GD2, EphA2, IL13R2α, EGFR, EGFR, ErbB2, etc) by qRT-PCR and western blots. Release criteria for the novel cell lines includes measures of sterility, endotoxin, mycoplasma, greater than 90% of cells have karyotypic abnormalities, and lack of replication competent retrovirus if this method of gene transfer is chosen. RNA-seq will be performed as described to identify specific commonly seen DIPG mutations (Fig 6A), and to validate the introduction of the target mutational burden. The algorithm developed herein is to seamlessly generate a cell product that is functionally analogous to the mouse counterparts.

### Example 5: Cancer Immunotherapy with APOBEC3B-Induced Heteroclitic Library Tumor Cell Vaccines and Immune Checkpoint Blockade

A freeze-thawed whole tumor cell vaccine prepared from B16 melanoma cells stably overexpressing human APOBEC3B treated established subcutaneous parental B16 tumors and, when combined with PD-1 checkpoint blockade, cured between 75%-100% of mice. Depletion of either CD4 or CD8 cells abrogated therapy. Moreover, T cells from mice treated with the vaccine and anti-PD1 produced high levels of interferon (IFN)-y when cocultured with parental unmodified B16 melanoma cells, correlating with therapeutic activity. Whole genome sequencing of B16 APOBEC3B overexpressing cell lines identified 301 C to T or G to A missense mutations unique to the APOBEC3B line. Using an *in silico* MHC binding affinity algorithm for peptides whose binding affinity for H2K^{b} or H2K^{d} was below a threshold of 500 nM, 10 high affinity APOBEC3B- induced heteroclitic peptides were identified and the ability of the B16 cells modified by APOBEC3B to prime T cells against these heteroclitic peptide candidates was experimentally validated *in vitro.* This approach was extended to an orthotopic brainstem model of High Grade Glioma. Thus, GL261 cells stereotactically implanted into the brainstem were significantly responsive to treatment with an APOBEC3B- modified GL261 vaccine in combination with anti-PD-1 checkpoint blockade and the priming of a T cell response to the APOBEC3B- modified GL261 vaccine was confirmed *in vitro.*

In summary, these data show that, when overexpressed in tumor cells, APOBEC3B generates a library of heteroclictic sequences, which primes both CD4 and CD8 T cells that have escaped central tolerance and which recognize both newly mutated antigens from the vaccine, as well as the corresponding unaltered self-epitopes expressed on the tumor cells. This approach represents a potent new method to develop neo-epitope based vaccine as a strategy for the treatment of, for example, pediatric high grade glioma.

### Example 6: An APOBEC3B Induced Library of Heteroclitic Neoepitopes Enhances Tumor Vaccines and Sensitizes T Cell Responses to Checkpoint Blockade

This example demonstrates that vaccination with tumor cell lines expressing human APOBEC3B primed tumor reactive T cells *in vivo* that were reactive against parental murine melanoma or glioma tumors, and that the efficacy of these T cells was amplified by ICB therapy. Candidate heteroclitic neoepitopes were identified using whole genome sequencing of APOBEC3B-altered vaccines and an *in silico* MHC-I binding screen. Following *ex vivo* validation, an individual neoepitope vaccine in combination with ICB was shown to partially recapitulate *in vivo* efficacy against unaltered tumors. Therefore, APOBEC3B can be used to enhance the immunogenicity of cell lysate vaccines via the introduction of heteroclitic epitopes.

### Results

### APOBEC3B overexpression promotes anti-tumor immune responses in the context of checkpoint inhibitor therapy

To test if neoepitopes introduced by APOBEC3B mutational events could be recognized by cognate T cells, B16 murine melanomas stably expressing the HSVtk suicide gene and either wild-type APOBEC3B (APOBEC3B^{WT}) or the catalytically inactive APOBEC3B mutant (APOBEC3B^{MUT}) were treated with two 5-day courses of ganciclovir (GCV) prodrug therapy, followed by anti-CTLA4 checkpoint inhibitor therapy (Fig. 7A). GCV therapy extended survival over untreated controls (Fig. 7B), however APOBEC3B^{WT} tumors escaped therapy more quickly than APOBEC3B^{MUT} tumors. (Fig. 7C). Sequencing of HSVtk in the escaped tumors revealed a consistent C-to-T, APOBEC3B signature mutation at base 21, resulting in a premature stop codon. Anti-CTLA4 therapy extended the median survival of mice bearing GCV-treated APOBEC3B^{MUT} tumors, confirming that HSVtk-mediated cell killing is immunogenic. However, anti-CTLA4 transformed the lesseffective GCV therapy for APOBEC3B^{WT} tumors into a sustained, curative treatment. These results suggested that while APOBEC3B overexpression can drive tumor escape from targeted small molecule therapy, it generated an increased mutational burden and allowed for recognition and rejection by the immune system in the context of immune checkpoint blockade.

### APOBEC3B overexpression improves tumor lysate vaccine efficacy when combined with immune checkpoint blockade

In order to capitalize upon the mutational burden generated by APOBEC3B expression without contributing to tumor evolution, an APOBEC3B-modified freeze-thawed tumor lysate vaccine was generated. Mice bearing subcutaneous B16 parental tumors were treated with B16 cell lysates retrovirally transduced to express either APOBEC3B^{WT} or APOBEC3B^{MUT}, followed by anti-PD1 or control immunoglobulin (IgG) (Fig. 8A). Mice treated with the tumor vaccine modified by overexpression of the APOBEC3B^{WT} exhibited delayed tumor growth (Fig. 8B) and prolonged survival (Fig. 8C) compared to mice treated with B16-APOBEC3B^{MUT} vaccines. This suggested that APOBEC3B overexpression can improve the immunogenicity of tumor cell vaccines. Only the B16-APOBEC3B^{WT} vaccine sensitized treated mice to immune checkpoint inhibition, with the addition of anti-PD1 therapy causing complete and sustained tumor regression out to 250 days in 75% of mice (Fig. 8C). This study was repeated with anti-PD1, anti-CTLA4, and combination checkpoint blockade, demonstrating similar results (Fig. 8D). B16 tumor lysate vaccines administered to mice bearing subcutaneous TC2 syngeneic prostate tumors were ineffective even in the context of APOBEC3B^{WT}/anti-PD1 therapy (Fig. 8C), suggesting the induced T cell response reflected the specific immunopeptidome of the vaccine. Symptoms, such as vitiligo, that would be associated with autoimmunity were not observed in any of these experiments.

### B16-APOBEC3B^{WT} vaccination elicits a CD4, CD8, and NK cell anti-tumor response

To identify the immune subsets involved in the vaccine's therapeutic efficacy, B16-APOBEC3B^{WT} vaccination and checkpoint blockade studies were repeated with depletion of CD4, CD8, or NK cells. Depletion of CD4, CD8, and NK cells significantly reduced the efficacy of the B16-APOBEC3B^{WT} and anti-PD1 therapy (Fig. 9A). When co-cultured *in vitro* with parental, unmodified tumor cells, splenocytes from mice which received B16-APOBEC3B^{WT} vaccines secreted significantly more interferon gamma (IFNy) than mice treated with B16-APOBEC3B^{MUT} (Fig. 9B). This IFNy secretion was increased even further when B16-APOBEC3B^{WT} vaccination was combined with ICB. When transferred into untreated mice bearing subcutaneous parental B16 tumors, CD8 T cells from mice treated with the B16-APOBEC3B^{WT} vaccine conferred partial protection against parental B16 tumors. In contrast, adoptive transfer of CD8 T cells from mice which received the control B16-APOBEC3B^{MUT} vaccine did not slow tumor growth compared to adoptive transfer of CD8 T cells from naive mice (Fig. 9C). Together, these results indicated that CD4, CD8, and NK cells were involved in B16-APOBEC3B^{WT} vaccine/checkpoint inhibitor combined therapy.

### APOBEC3B overexpressing tumor cell vaccine confers therapeutic efficacy in an aggressive brainstem glioma model

To validate that APOBEC3B-modified vaccination efficacy is not limited to melanomas or subcutaneous tumors, the APOBEC3B-modified tumor vaccine platform was tested in an aggressive brainstem glioma model. Mice bearing brainstem-implanted GL261 tumors were treated with an APOBEC3B^{WT} or APOBEC3B^{MUT}-modified GL261 vaccine (Fig. 10A). Mice treated with the GL261-APOBEC3B^{WT}-modified vaccine survived significantly longer than untreated controls (Fig. 10B). The GL261-APOBEC3B^{WT}-modified vaccine in combination with anti-PD1 doubled median survival, though when controlling for multiple comparisons it did not reach statistical significance when compared to untreated controls. However, the combination of anti-CTLA4 and anti-PD1 therapy with the GL261-APOBEC3B^{WT} vaccine significantly improved survival compared to mice treated with control immunoglobulin, with all mice surviving past 40 days.

To determine if the T cell response raised by the APOBEC3B-modified vaccine crossreacted onto unmodified parental tumor cells, splenocytes from treated mice were co-cultured with parental GL261 cell *in vitro.* Splenocytes isolated from mice that received the GL261-APOBEC3B^{WT} vaccine and ICB secreted significantly increased levels of IFNγ after 72 hours of co-incubation with parental GL261 tumors compared to mice receiving only the GL261-APOBEC3B^{WT} vaccine (Fig. 10C). These results showed that the APOBEC3B^{WT}-modified vaccine and ICB strategy is effective against a second tumor type and retains efficacy in an immunologically distinct site like the brainstem.

### APOBEC3B induces neoepitopes with predicted heteroclitic activity

A screen of the B16tk-APOBEC3B^{WT}-modified, VSV-escaped population, compared to the B16tk parental cells using whole genomic sequencing identified 1,048,576 mutations unique to the B16-APOBEC3B^{WT} overexpressing cells. (SRA Submission: SRP159367). Of those, 237 contained C to T or G to A transitions that lead to missense mutations and were consistent with APOBEC3B mutational activity (Table 1; Figure 16). These mutational sites were converted into 21-amino acid sequences, with 10 amino acids flanking the mutational site (Table 2; Figure 17). 8-10mer peptides were generated from these sequences with the mutated amino acid in every position from the amino terminal to the carboxy terminal end, and these peptides were filtered through an MHC binding affinity algorithm (Table 3). To determine if a proportion of APOBEC3B-induced neoepitopes in the vaccine cells could also act as heteroclitic peptides, the potential neoepitopes were screened for differential binding to MHC Class I. Thresholds were set for binding affinity to either H2K^{b} (Fig. 11A dark grey and dark blue) or H2D^{b} (Fig. 11A light grey and light blue), such that wild-type peptide binding was above 500 nM, while APOBEC3B-mutated peptide binding was below 500 nM. By refining this list using the EMBL-EBI Expression Atlas eight candidate proteins were identified that were expressed in the skin: Ahctfl, C77080, Csde1, Fcgbp, Plbd2, Smc4, Stag2, and Xpo1 (which contained three candidate peptide sequence variants) (Fig. 11A).

**Table 3. MHC binding affinities for heteroclitic neoepitope candidates. Binding affinities for the top ten heteroclitic neoepitope candidates with differential binding affinity to either H-2-Db or H-2-Kb and also expressed in the skin.**

| Gene | variant no | HLA | peptide (SEQ ID NO) | pep ID | Affinity (nM) | peptide_MT (SEQ ID NO) | Affinity(nM) MT | differentialMTsubtractWT | logFCMT WT |
|---|---|---|---|---|---|---|---|---|---|
| Ahctf1 | 14 | H-2-Db | NSAANLRFV (SEQ ID NO:3) | WT_A578T_Ahctf1 | 615.66 | NSATNLRFV (SEQ ID NO:4) | 469.31 | -146.35 | 0.762287626 |
| C77080 | 31 | H-2-Kb | ASFIFSKG (SEQ ID NO:5) | WT_A903T_C77080 | 602.55 | TSFIFSKG (SEQ ID NO:6) | 491.96 | -110.59 | 0.816463364 |
| Csde1 | 86 | H-2-Kb | MSFDPNLL (SEQ ID NO:7) | WT_P5S_Csde1-00 | 587.81 | MSFDSNLL (SEQ ID NO:8) | 345.25 | -242.56 | 0.587349654 |
| Fcgbp | 111 | H-2-Kb | RSEELCPL (SEQ ID NO:9) | WT_L715F_Fcgbp- | 4640.94 | RSEEFCPL (SEQ ID NO:10) | 129.99 | -4510.95 | 0.028009412 |
| Plbd2 | 299 | H-2-Kb | SSGGWAARA (SEQ ID NO:11) | WT_A16V_Plbd2-0 | 4202.63 | SSGGWAARV (SEQ ID NO:12) | 440.6 | -3762.03 | 0.104839113 |
| Smc4 | 338 | H-2-Db | SSVIDEISV (SEQ ID NO:13) | WT_D767N_Smc4-0 | 4439.13 | SSVINEISV (SEQ ID NO:14) | 56.05 | -4383.08 | 0.012626348 |
| Stag2 | 356 | H-2-Kb | VRLKCLTAL (SEQ ID NO:15) | WT_L345F_Stag2- | 1084.75 | VRFKCLTAL (SEQ ID NO:16) | 256.26 | -828.49 | 0.236238765 |
| Xpo1 | 404 | H-2-Kb | TLVYLTHL (SEQ ID NO:17) | WT_L464F_Xpo1-2 | 969.17 | TLVYFTHL (SEQ ID NO:18) | 47.79 | -921.38 | 0.049310235 |
| Xpo1 | 404 | H-2-Kb | VYLTHLDYV (SEQ ID NO:19) | WT_L464F_Xpo1-2 | 638.67 | VYFTHLDYV (SEQ ID NO:20) | 198.86 | -439.81 | 0.311365807 |
| Xpo1 | 404 | H-2-Kb | RETLVYLTHL (SEQ ID NO:21) | WT_L464F_Xpo1-2 | 782.12 | RETLVYFTHL (SEQ ID NO:22) | 99.65 | -682.47 | 0.127410116 |

### In vitro validation of APOBEC3B-induced neoepitopes

To evaluate experimentally whether the B16-APOBEC3B^{WT} vaccine was able to prime T cells against the *in silico-predicted* neoepitope candidates, B16 cell lines were generated that transiently overexpressed the 10 wild type (B16-WT sequence) peptides or the mutant neoepitope peptides (B16-APOBEC3B modified sequence). Expression constructs contained the ten wild type or neoepitope peptides separated by Ala-Ala-Tyr (AAY) spacers between each peptide and tagged with an N-end degron motif to increase MHC Class I presentation (Fig. 11B). Splenocytes from unvaccinated or B16-APOBEC3B^{MUT} vaccine treated mice showed no IFNy recall response to cells overexpressing B16-WT peptides or B16-APOBEC3B modified peptides (Fig. 11C). However, splenocytes from mice treated with the B16-APOBEC3B^{WT} vaccine and ICB showed a strong recall response against cells expressing the B16-APOBEC3B modified peptides. They also showed a recall response against B16-WT sequence cells. This suggested that one or more of the 10 candidate epitopes were also heteroclitic epitopes generated within the B16-APOBEC3B^{WT} vaccine, such that it was able to activate T cells to cross react against wild type epitopes expressed on parental B16 cells. To identify which of these predicted neoepitopes may be targets for T cell responses in the C57Bl/6 mice, expression constructs were created for each of the individual neoepitopes. Splenocytes from naive mice or from mice vaccinated with the B16-APOBEC3B^{MUT} vaccine did not generate a recall response against parental B16 tumor cells *in vitro* (Fig. 11D). In contrast, as before, splenocytes from mice vaccinated with B16-APOBEC3B^{WT} cells showed a significant recall response against parental B16 cells (Fig. 11D), suggesting that T cell responses against those neoepitopes in the vaccine, which also serve as heteroclitic epitopes, could cross react against wild type epitopes expressed on the parental, unmodified cells. In particular, splenocytes from mice vaccinated with B16-APOBEC3B^{WT} cells also showed a significant enhancement of the recall response against parental B16 cells overexpressing the mutated cold shock domain-containing protein E1 (CSDE1) peptide (Fig. 11D: CSDE1*). Over-expression of none of the remaining 9 potential predicted APOBEC3B-modified neoepitopes enhanced the recall response above that seen in response to parental B16 cells themselves (Fig. 11D). These results confirmed that vaccination with B16-APOBEC3B^{WT} cells induced T cell responses against parental B16 tumors and suggest that an APOBEC3B-mutated neoepitope within CSDE1 may function as a heteroclitic epitope.

In order to validate that the putative CSDE1 heteroclitic epitope was indeed present in the vaccine preparation, the CSDE1 gene was sequenced in B 16-APOBEC3B^{WT} vaccine cells used in Figures 8-10. Consistent with the lack of APOBEC3B deaminase activity of the APOBEC3B^{MUT} construct, B16 parental and B16 APOBEC3B^{MUT} cell vaccines contained only the wild type ATGAGCTTTGATCCA (SEQ ID NO: 1) sequence (Fig. 12A, 12B). However, the vaccine preparation contained a mixed population of cells carrying either the wild type ATGAGCTTTGATCCA (SEQ ID NO: 1) sequence, as found homogeneously in the parental B16 and B16-APOBEC3B^{MUT} vaccine populations or the mutated ATGAGCTTTGATTCA (SEQ ID NO:2) sequence (Fig. 12C), which encodes the heteroclitic CSDE1 neoepitope (Fig. 11D). It was further validated that the CSDE1 mutation is a reproducible and consistent target of APOBEC3B activity in B16 cells in two additional vaccine preparations (Fig. 13).

### The CSDE1 heteroclitic epitope primes T cells which provide therapy against B16 tumors

It was then sought to evaluate the strength of the CSDE1 epitope and its relative contribution to the *in vivo* vaccine efficacy. B16 cells transfected with the mutant CSDE1 epitope (CSDE1*) or the wild-type parental epitope (CSDE1) were used as a vaccine in combination with anti-PD1 to treat parental B16 tumors in the flank or brainstem. Mice vaccinated with B16 cells expressing CSDE1 succumbed to disease at the same time as untreated controls (Fig. 14A, Fig. 14B). The single CSDE1* epitope achieved a partial response, whereas the B16-APOBEC3B^{WT} vaccine was significantly more effective, highlighting the value of vaccination with a wide range of antigens to prevent tumor escape. When splenocytes from these mice were cocultured with either parental B16 or TC2 prostate tumor cells expressing either CSDE1 or CSDE1* epitopes, only mice vaccinated with B16-CSDE1* and treated with ICB showed an IFNy recall response against parental B16 cells (Fig. 14C). In the context of an irrelevant TC2 tumor, mice vaccinated with B16-CSDE1* reacted against both CSDE1* and CSDE1 epitopes, thereby confirming that the mutant CSDE1 acts as a heteroclitic peptide.

### Materials and Methods

### Study Design

This study was designed to evaluate the use of an APOBEC3B modified vaccine to prolong survival of tumor bearing mice and investigate its synergy with immune checkpoint blockade. Seven mice per group were used for each experiment to achieve statistical power to make multiple comparisons. Mice were randomized at time of tumor implantation and tumors were measured by a single blinded individual.

### Cell lines

B16.F1 murine melanoma cells were obtained from the ATCC. B16TK cells were derived from a B16.F1 clone transfected with a plasmid expressing the Herpes Simplex Virus thymidine kinase (HSV-1 TK) gene in 1997/1998. Following stable selection in 1.25 µg/mL puromycin, these cells were shown to be sensitive to Ganciclovir (Cymevene) at 5 µg/mL (see, *e.g.,* Le *et al.,* 2010 *Cancer J* 16:304; Lipson *et al.,* 2015 *J Transl Med* 13:214; Dyall *et al.,* 1998 *J Exp Med* 188:1553). B16TK cells were grown in DMEM (HyClone, Logan, UT, USA) + 10% FBS (Life Technologies) + 1.25 µg/mL puromycin (Sigma) until challenge. TRAMP-C2 (TC2) cells are derived from a prostate tumor that arose in a TRAMP mouse and were characterized as described elsewhere (see, *e.g.,* Foster et al., 1997 Cancer Res 57:3325; Gingrich et al., 1996 Cancer Res 56:4096). Cell lines were authenticated by morphology, growth characteristics, PCR for melanoma specific gene expression (gp100, TYRP-1 and TYRP-2) and biologic behavior, tested mycoplasma-free, and frozen. Cells were cultured less than 3 months after thawing. Cells were tested for mycoplasma using the MycoAlert Mycoplasma Detection Kit (Lonza Rockland, Inc. ME, USA).

### Immune cell activation

Spleens and lymph nodes were immediately excised from euthanized C57Bl/6 or OT-I mice and dissociated *in vitro* to achieve single-cell suspensions. Red blood cells were lysed with ACK lysis buffer (Sigma-Aldrich) for 2 minutes. Cells were resuspended at 1 × 10⁶ cells/mL in Iscove's Modified Dulbecco's Medium (IMDM; Gibco) supplemented with 5% FBS, 1% penicillin-streptomycin, 40 µmol/L 2-Mercaptoethanol. Cells were co-cultured with target cells as described in the text. Cell-free supernatants were then collected 72 hours later and tested for IFNy (Mouse IFNy ELISA Kit; OptEIA, BD Biosciences) production by ELISA as directed in the manufacturer's instructions.

### APOBEC3 overexpression and vaccine preparation

B16TK cells were transduced with a retroviral vector encoding either full length functional APOBEC3B (APOBEC3B^{WT}) or a mutated, catalytically inactive form of APOBEC3B (APOBEC3B^{MUT}) as a negative control (see, *e.g.,* Pak et al., 2011 J Virol 85:8538). 48 hours post transduction with either pBABE-Hygro APOBEC3B^{WT} or pBABE-Hygro APOBEC3B^{MUT} viruses, bulk populations of cells were selected in hygromycin for 2 weeks and used for experiments. Overexpression of APOBEC3B was confirmed by both Western Blot (using a rabbit monoclonal anti-human APOBEC3B (184990, Abcam, San Francisco, CA)) and qrtPCR as previously described elsewhere (see, *e.g.,* Huff et al., 2018 Mol Ther Oncolytics 11: 1-13). It was observed that over-expression of APOBEC3B is toxic in that elevated levels of APOBEC3B are seen within 72 hours post-transfection/transduction and return to similar levels to that seen in parental unmodified cells. Without being bound by theory, this may be because mutagenesis by APOBEC3B is tolerable to the cell up to a certain threshold, and then in cells where critical mutations are induced, this can be lethal. In other cells, overexpression of APOBEC3B may not reach the threshold, or mutations may not be induced in critical genes, allowing those cells to survive carrying the APOBEC3B-induced mutations.

B16 or GL261 cells either left untreated, or modified to over-express either functional APOBEC3B (B16-APOBEC^{WT}) or the non-functional APOBEC3B MUT protein (B16-APOBEC3B^{MUT}) were expanded in T175 flasks. At 80-90% confluency, cells were trypsinized and washed three times in PBS (HyClone). Aliquots of 5x10⁷ cells were resuspended in a volume of 1 mL PBS and then freeze-thawed for three cycles in liquid nitrogen. 100 µl of these freeze-thawed vaccine preparations were administered intra-peritoneally (i.p.) to mice (the equivalent of 10⁶ cells per injection).

### In vivo experiments

All *in vivo* studies were approved by the Institutional Animal Care and Use Committee at Mayo Clinic. 6-8 week old female C57BL/6 mice were purchased from Jackson Laboratories (Bar Harbor, Maine). Mice were challenged subcutaneously with 2x10⁵ B16TK murine melanoma cells, their APOBEC3B^{WT} or APOBEC3B^{MUT} derivatives, parental B16 murine melanoma cells, or TC2 murine prostate carcinoma cells in 100 µL PBS. Alternatively, 5x10⁴ GL261 cells or 1x10⁴ B16 cells were implanted in 2 µL intra-cranially into the brainstem. Subcutaneous tumors were treated with a two week course of GCV (50 mg/kg) administered i.p. daily; or with a 5 day course of B16-APOBEC3B^{WT} or B16-APOBEC3B^{MUT} cell vaccines as described in the text. Subcutaneous tumors were measured 3 times per week, and mice were euthanized when tumors reached 1.0 cm in diameter. GL261 tumor cells were stereotactically implanted into the brainstems of C57Bl/6 mice as described elsewhere (see, *e.g.,* Caretti et al., 2011 Brain Pathol 21:441). Intracranial tumors were treated with a 5 day course of GL261- or B16-APOBEC3B^{WT} or APOBEC3B^{MUT} cell vaccines as described in the text. Mice were sacrificed upon emergence of neurological symptoms or weight loss.

### Immune cell depletions and checkpoint inhibition

To deplete specific immune subsets, mice were treated with intraperitoneal (i.p.) injections (0.1 mg per mouse) of anti-CD8 (Lyt 2.43, BioXCell), anti-CD4 (GK1.5, BioXCell), anti-NK (anti-asialo-GM-1, Cedarlane) and IgG control (ChromPure Rat IgG, Jackson ImmunoResearch) at day 4 after tumor implantation and then weekly thereafter. FACS analysis of spleens and lymph nodes confirmed subset specific depletions. For immune checkpoint blockade, mice were treated intravenously with anti-PD1 (0.25 mg; catalog no. BE0146; Bio X Cell), anti-CTLA-4 (0.1 mg; catalog no. BE0164; Bio X Cell), anti-asialo GM1 (0.1 mg; catalog no. CL8955; Cedarlane), or isotype control rat IgG (catalog no. 012-000-003; Jackson ImmunoResearch) antibody at times described in each experiment.

### Next Generation Sequencing

DNA for whole genome sequencing was prepared from B16tk cells that overexpressed either APOBEC3B^{WT} or parental, unmodified cells. These cells were initially generated from an experiment investigating the role of APOBEC3B overexpression on the generation of resistance to Vesicular Stomatitis Virus (VSV) oncolysis (see, *e.g.,* Huff et al., 2018 Mol Ther Oncolytics 11: 1-13). Following three rounds of infection with VSV, genomic DNA was isolated from APOBEC3B expressing cells resistant to infection or parental, mock-infected cells. Five hundred nanograms of each sample was prepared using an Ultra kit (New England Bio) and underwent paired end 150bp sequencing on the Illumina HiSeq 4000 by Mayo's Genome Analysis Core. Sequences were aligned to the mm10 C57Bl/6 genome by the Mayo's Bioinformatics Core Facility and mutational changes were detected between the two samples. These mutations contained 301 C to T or G to A missense mutations which were unique to the APOBEC3B^{WT} line. These coding variants were filtered through NET MHC 2.0 binding affinity algorithm to identify octamer or nonamer peptides whose binding affinity for H2K^{b} or H2K^{d} was below a threshold of 500 nM, and whose their corresponding wild-type peptides had a binding affinity to the same molecules above 500 nM. A list of 36 candidates was further refined using the EMBL-EBI Expression Atlas for expression in skin tissue to identify 10 high affinity APOBEC3B- induced heteroclitic peptides.

### APOBEC3B-modified Epitope Expression

cDNAs encoding either wild type (un-mutated) or APOBEC3B-mutated 9 mer peptides derived from the screen described above were synthesized and cloned into the plasmid pcDNA3.1+P2A-eGFP (Genescript), where the cDNA is expressed from a CMV promoter and co-expressed with an eGFP protein. pcDNA3.1-EPITOPE+P2A-eGFP plasmids were transfected into B16 cells using Lipofectamine. Cultures in which transfection of over 50% of cells was confirmed by GFP analysis 48-72 hours post transfection were used either as targets in immune activation assays (above) or were expanded in T175 flasks. At 80-90% confluency, cells were trypsinized and washed three times in PBS. Aliquots of 5x10⁷ cells were re-suspended in a volume of 1 mL PBS and then freeze-thawed for three cycles in liquid nitrogen. 100 µl of these freeze-thawed vaccine preparations were administered intra-peritoneally to mice (the equivalent of 10⁶ cells per injection).

### Statistics

Survival curves were analyzed by the Log-Rank test with Holm-Bonferroni correction for multiple comparisons. Student's T tests, one way ANOVA and two way ANOVA were applied for *in vitro* assays as appropriate. Statistical significance was set at p<0.05 for all experiments. In summary, when overexpressed in tumor cells, APOBEC3B generated a library of heteroclitic sequences which primed both CD4 and CD8 T cells that had escaped central tolerance and which recognized both newly mutated antigens from the vaccine, and the corresponding unaltered self epitopes expressed on the tumor cells. These results validate the methodology to identify new candidate tumor antigens which may be targeted by vaccination.

### Example 7: APOBEC3 Overexpression

### Materials and Methods

### Plasmid construction

Both wild-type (WT) human APOBEC3B (NM_004900.4) and an APOBEC3B catalytic mutant (CM) which contains the E68A/E255A mutations were synthesized as codon-optimized Gene Blocks (Integrated DNA Technologies) with a C-terminal HA-tags. Both constructs were then cloned into a lentiviral vector, pSIN-CSGW-PGKpuro by way of Bcl1/BamH1-hybrid and Xho1/Sal1 hybrid sites. Additionally, both WT and CM APOBEC3B were cloned into pAAV-MCS (Stratagene) by way of Bcl1/BamH1 and Xho1 sites. All sequences were validated by Sanger sequencing (GeneWiz) using primers that flank the coding region.

### Vector productions

Human embryonic kidney 293T cells maintained in Dulbecco's modified Eagle's medium with 10% fetal bovine serum. All plasmid transfections were done using Fugene6 (Promega). The AAV vector stocks were produced in 293T cells using the helper-free transfection method according to the manufacturer's protocol (Stratagene). AAV6 capsidexpressing plasmid pRep2Cap6 (Stratagene) were used for AAV6 vector production. Briefly, 293T cells were transfected with three plasmids, including pHelper (Stratagene), Rep2Cap6, and a transfer vector plasmid (pAAV-CMV-EmGFP, pAAV-CMV-hA3B-WT, pAAV-CMV-hA3B-CM). Three days after transfection, AAV6 vector-producing 293T cells were harvested for vector purification. The cells were lysed by three rounds of freeze-and-thaw, followed by ultracentrifuge concentration (62,500 rpm for 2 hours) through Optiprep Density Gradient Medium (Sigma). The resulting AAV6 vectors were desalted and further concentrated using Amicon Ultra-15 100k filtration (Amicon). The titers (genomic copy numbers/mL) of concentrated AAV6 vector stocks were determined by Q-PCR using plasmid DNA standards and AAV genomic sequence-specific primers and fluorescent probe.

HIV-based lentiviral vectors were generated by three plasmid transfection (p8.91QV, VSV-G and pHR SIN expression plasmid) in 293T cells. Cells were cultured for three days, the culture media was then collected, passed through a 0.45 µm filter and concentrated down by ultra-centrifugation at 25,000 rpms for 1.5 hours. The resulting viral pellets were resuspended in PBS and titered in 293T cells by FACS either GFP or HA expressions (refer to flow cytometry section for more details).

### Cell-lines

The human DIPG neurosphere cell-lines, SOH and SF7761 (Millipore Cat# SCC126), were cultured in tumor stem medium (TSM) containing Neurobasal-A Media (Invitrogen, Cat#10888-22) and D-MEM/F-12 media (Invitrogen, Cat#11330-032) at a 1:1 ratio. This was further supplemented with 1 M HEPES, 100 mM MEM Sodium Pyruvate, 10 mM MEM Non-Essential Amino Acids, GlutaMax-I supplement (1X), and Antibiotic-Antimycotic (1X). Working TSM was prepared freshly, by further supplementing in B-27 supplement Minus Vitamin A (Invitrogen, Cat# 12587-010), 20 ng/ml H-EGF (Shenandoah Biotech, Cat# 100-26), 20 ng/ml H-FGF (Shenandoah Biotech, Cat# 100-146), 10 ng/ml H-PDGF-AA (Shenandoah Biotech, Cat# 100-16), 10 ng/ml H-PDGF-BB (Shenandoah Biotech, Cat# 100-18) and 2 µg/ml Heparin solution (StemCell Tech, Cat#07980). Cells were maintained at 37°C in a 5% CO₂ humidified incubator.

### DIPG Transductions

SOH or SF7761 cells were dissociated in TrypLE Express (Invitrogen, Cat# 12605010), counted and seeded in working TSM at 1e5 cells/well of a 48-well plate. Within 4-hours post-dissociation, cells were transduced with either AAV6 vectors at MOI of 10,000 genome copies/cell or lentiviral vectors at a MOI of 1 or 3. Cells were harvested at indicated times for subsequent analysis.

### Flow cytometry

Cells were collected, dissociated into single-cell suspensions by incubation in TrypLE Express for 10 minutes at 37°C. Following incubation, cells were briefly pelleted, washed in PBS, pelleted and re-suspended PBS containing Zombie NIR solution (Biolegend, Cat# 423105). Cells were protected from light and incubated at room temperature for 15 minutes. Cells were then washed in PBS, fixed and permeabilized with BD Cytofix/Cytoperm kit (BD Biosciences, Cat#554714) at room temperature for 20 minutes. Cells were maintained in 1x perm/wash buffer (BD Biosciences), blocked with 5% fetal bovine serum for at least 1 hour before applying primary antibodies. All antibodies were diluted in 1x perm/wash buffer and incubated overnight at 4°C with PE anti-HA.11 Epitope Tag Antibody (Biolegend, Cat#901518) at 1:200 dilution. Following incubation, cells were washed 2x in PBS and analyzed on CantoX flow cytometer (BD Biosciences). Analysis of the results was performed using FlowJo software.

### RT-qPCR

Roughly 1e5-2e5 cells were directly lysed in RLT buffer (Qiagen RNeasy Plus Kit, Cat#74134) supplemented with beta-mercaptoethanol. RNA was purified based on manufactures protocol. 100 ng of total RNA was converted into cDNA by SuperScript III Reverse Transcriptase (Invitrogen, Cat# 18080044), dNTP solutions (Thermo Fisher Scientific, #18427013), RNaseOUT (Thermo Fisher Scientific,#10777019) and Random Hexamer (Thermo Fisher Scientific, #48190011).

SYBR-green quantitative PCR was conducted using qPCR Master mix kit (MIDSCI, Cat #BEQPCR-IC). Primer sequences used to detect the codon-optimized hA3B transcripts were generated by primer-blast software (NIH):
hA3B Codon Opt Forward: AAGTGAAAATCAAGCGCGGG (SEQ ID NO:569)
hA3B Codon Opt Reverse: CAATTTGGCGACACAGTCGG (SEQ ID NO:570)

PrimeTime primer assays were purchased from IDT for the detection of endogenous hA3B transcripts and for housekeeping reference gene detection, Hs.PT.58.45417356 and Hs.PT.58v.45621572, respectively.

The PCR conditions were 95°C for 10 minutes enzyme activation, 95°C for 15 seconds denaturation, 60°C for 60 seconds annealing and extension, and overall 40 cycles were performed.

### DNA cytosine deaminase oligo cleavage assay.

293T cells were seeded at 1e5 cells/well and transduced with Lenti-hA3B-Wt or CM 4 hours later. The cells were allowed to grow for 72-hours before passage and puromycin selection (3.33 µg/ml). At day 7 post-infection, the APOBEC3B expressing 293Ts were dissociated in Trypsin and counted. Whole-cell extracts were prepared from 1e6 cells in 200 µl of HED buffer (25 mM HEPES, 5 mM EDTA, 10% Glycerol, 1 mM DTT and 1x EDTA-free Protease inhibitor (Roche)). Lysates were kept on ice, sonicated and centrifuged at 4°C for 30 minutes. The resulting supernatant was collected and used immediately for deamination reactions. The 20 µl deamination reaction contained 1 µl 4 pM 3'FITC labeled 43-mer oligo, 0.25 µl UDG (NEB), 0.25 µl RNAse A, 2 µl 10x UDG buffer (NEB) and 16.5 µl whole-cell lysates. This was mixed and incubated at 37°C for 1 hour. 2 µl of 1M NaOH was added, and the mixture was heated at 95°C for 10 minutes, cooled briefly, and then 22 µl of 2x Formamide loading buffer was added. 5 µl of each reaction were loaded on to a 15% TBE Urea gel. Electrophoresis at 100 volts for roughly one hour was performed. Gels were imaged on a Typhoon gel imager (GE Healthcare).

### Human T cell activation assays

Human donor PBMC were sorted into CD3+ cells and CD14+ monocytes. Monocytes were differentiated into immature DC with GM-CSF (800 U/ml) and IL-4 (1000 U/ml) for 7 days. DC were then loaded with cell lysates (from PG1 human paediatric DIPG cells, human Mel888 or PG-1-APOBEC3B transduced PG-1 cells) at an equivalent of ~2x10⁵ tumour cells per 10⁴ DC on days 7, 8, and 9 and grown in GM-CSF, IL-4, TNF-α (1100 U/ml), IL-1β (1870 U/ml), and IL-6 (1000 U/ml), to generate mature DC actively presenting tumor associated antigens of PG1 cells. On day 10, autologous donor CD3+ T cells were thawed and co-cultured with mature, loaded DC at an E:T ratio of 10:1. 10 days later, CD3+ T cells were purified from the culture by magnetic beads and labelled with Cell Tracker Violet. These expanded CD3+ T cells were then co-cultured with autologous 10 day mature DC loaded three times with PG1 lysates (as above) as targets at a ratio of 10 (T):1 (DC). 72 hours later, proliferation of the CD3+ T cells was measured using flow cytometry for dilution of Cell trace violet and levels of IFN-g released into the supernatants were measured by ELISA.

### Immunostaining

PC3, SW80 and H226 cells were transduced with a lentiviral vector (MOI 1) or AAV6 (1e4 gc/cell) expressing the HA-tagged WT or catalytically inactive mutant human APOBEC3B. 48 hours post transduction, cells were reseeded in chamber slides and 24h later fixed with 4% paraformaldehyde, permeabilized in BD Cytofix Cytoperm and blocked with 5% FBS in PBS. Cells were stained with anti-rat HA and donkey anti-rat AF594; rabbit anti-H2AX and donkey anti-rabbit FITC. Nuclei were counterstained by 4',6-diamidino-2-phenylindole (DAPI). Stained cells were observed through a Zeiss LSM 780 confocal laser scanning microscope, and the images were analyzed by using the Zeiss imaging software.

### Results

Lentiviral vectors and AAV vectors were used to express either a wild type (WT) or catalytically inactive mutant (CM) human APOBEC3B. The APOBEC3B was tagged with HA in order to track the expression of the protein. The expression of the transgene was confirmed by flow cytometry using an anti-HA antibody (Figure 18) and by qRT-PCR (Figure 19) in two different DIPG cell lines.

The cytidine deaminase function of the transgene expressed from the lentivirus was demonstrated using a deaminase assay (Figure 20). A fluorescently labeled probe which contained an APOBEC3B site was incubated with lysate from transduced cells. Mutation of the probe by APOBEC3B in the presence of Uracil DNA Glycosylase (UDG) allows for alkali cleavage and the detection of the smaller cleavage fragment. Transduction of 293T cells or a DIPG cell line with the hA3B-WT, but not the catalytically inactive mutant, resulted in the detection of the cleavage product.

To demonstrate the functionality of the vector to increase the immunogenicity of a cell substrate in a DC vaccine, a lentiviral vector was used in an *in vitro* priming and recall assay (Figure 21). Dendritic cells were isolated from a healthy donor and differentiated *in vitro* from monocytes and loaded with lysate from the SOH cell line, or lysate from lentivirus APOBEC3B transduced cells. DCs were used to prime autologous T cells for 5 days, and then fresh DCs loaded with the parental SOH cell line were used to restimulate the *in vitro* primed T cells. Consistent with the results from the murine studies, the recall response against the parental SOH line was amplified if the T cells were primed with APOBEC3B modified lysate rather than the lysate transduced with the control GFP expressing virus. Furthermore, the recall response was enhanced in the presence of anti-PD1 antibodies.

APOBEC3B expressing lentivirus and AAV vectors can be used to transduce a variety of other cancer cell types, including prostate cancer, colon cancer, and lung cancer (Figure 24).

Together these results demonstrate that APOBEC3B modified cancer cell vaccines can be used for a broad array of cancer etiologies.

## Claims

1. A composition comprising one or more tumor cells comprising a viral vector encoding APOBEC3B polypeptides wherein the said one or more tumor cells have been loaded into or fed to a dendritic cell, wherein said viral vector is selected from an adenoviral vector and a retroviral vector, wherein said one or more tumor cells express said APOBEC3B polypeptides and generate a library of mutated tumor antigens within said one or more tumor cells, wherein said tumor antigens comprise one or more heteroclitic mutations, wherein said dendritic cell presents said library of mutated tumor antigens.

2. The composition of claim 1, wherein said APOBEC3B polypeptides are human APOBEC3B polypeptides.

3. The composition of any one of claims 1-2, wherein said tumor antigens comprise one or more self antigens.

4. The composition of any one of claims 1-2, wherein said tumor antigens comprise one or more non-self antigens.

5. The composition of any one of claims 1-2, wherein said tumor antigens comprise one or more neoantigens.

6. The composition of any one of claims 1-5, wherein said one or more tumor cells comprise autologous cells.

7. The composition of any one of claims 1-5, wherein said one or more tumor cells comprise allogeneic cells.

8. A composition of any one of claims 1-7 for use in a method for treating a cancer in a mammal, said method comprising administering said composition to said mammal.

9. The composition for use of claim 8, wherein said one or more tumor cells are obtained from a mammal to be treated.

10. The composition for use of claim 8, wherein said one or more tumor cells are obtained from a donor mammal.

11. The composition for use of any one of claims 8-10, wherein said mammal is a human.

12. The composition for use of any one of claims 8-11, wherein the cancer is selected from glioma, melanoma, prostate cancer, and lung cancer.

## Patentansprüche

1. Zusammensetzung, umfassend eine oder mehrere Tumorzellen, umfassend einen viralen Vektor, der APOBEC3B-Polypeptide codiert, wobei die eine oder die mehreren Tumorzellen in eine dendritische Zelle geladen oder in eine dendritische Zelle eingespeist wurden, wobei der virale Vektor aus einem adenoviralen Vektor und einem retroviralen Vektor ausgewählt ist, wobei die eine oder die mehreren Tumorzellen die APOBEC3B-Polypeptide exprimieren und eine Bibliothek mutierter Tumorantigene innerhalb der einen oder mehreren Tumorzellen erzeugen, wobei die Tumorantigene eine oder mehrere heteroklitische Mutationen umfassen, wobei die dendritische Zelle die Bibliothek mutierter Tumorantigene präsentiert.

2. Zusammensetzung nach Anspruch 1, wobei die APOBEC3B-Polypeptide humane APOBEC3B-Polypeptide sind.

3. Zusammensetzung nach einem der Ansprüche 1 bis 2, wobei die Tumorantigene ein oder mehrere Selbstantigene umfassen.

4. Zusammensetzung nach einem der Ansprüche 1 bis 2, wobei die Tumorantigene ein oder mehrere Nicht-Selbstantigene umfassen.

5. Zusammensetzung nach einem der Ansprüche 1 bis 2, wobei die Tumorantigene ein oder mehrere Neoantigene umfassen.

6. Zusammensetzung nach einem der Ansprüche 1 bis 5, wobei die eine oder die mehreren Tumorzellen autologe Zellen umfassen.

7. Zusammensetzung nach einem der Ansprüche 1 bis 5, wobei die eine oder die mehreren Tumorzellen allogene Zellen umfassen.

8. Zusammensetzung nach einem der Ansprüche 1 bis 7 zur Verwendung in einem Verfahren zur Behandlung eines Krebses bei einem Säuger, wobei das Verfahren das Verabreichen der Zusammensetzung an den Säuger umfasst.

9. Zusammensetzung zur Verwendung nach Anspruch 8, wobei die eine oder die mehreren Tumorzellen aus einem zu behandelnden Säuger erhalten werden.

10. Zusammensetzung zur Verwendung nach Anspruch 8, wobei die eine oder die mehreren Tumorzellen aus einem Spendersäuger erhalten werden.

11. Zusammensetzung zur Verwendung nach einem der Ansprüche 8 bis 10, wobei es sich bei dem Säuger um einen Menschen handelt.

12. Zusammensetzung zur Verwendung nach einem der Ansprüche 8 bis 11, wobei der Krebs ausgewählt ist aus Gliom, Melanom, Prostatakrebs und Lungenkrebs.

## Revendications

1. Composition comprenant une ou plusieurs cellules tumorales comprenant un vecteur viral codant pour des polypeptides APOBEC3B, dans laquelle ladite ou lesdites cellules tumorales ont été introduites dans une cellule dendritique ou présentées à celle-ci, ledit vecteur viral est choisi parmi un vecteur adénoviral et un vecteur rétroviral et ladite ou lesdites cellules tumorales expriment lesdits polypeptides APOBEC3B et génèrent une bibliothèque d'antigènes tumoraux mutés à l'intérieur de ladite ou desdites cellules tumorales, lesdits antigènes tumoraux comprenant une ou plusieurs mutations hétéroclites, ladite cellule dendritique présentant ladite bibliothèque d'antigènes tumoraux mutés.

2. Composition selon la revendication 1, dans laquelle lesdits polypeptides APOBEC3B sont des polypeptides APOBEC3B humains.

3. Composition selon l'une quelconque des revendications 1 et 2, dans laquelle lesdits antigènes tumoraux comprennent un ou plusieurs autoantigènes.

4. Composition selon l'une quelconque des revendications 1 et 2, dans laquelle lesdits antigènes tumoraux comprennent un ou plusieurs antigènes non-soi.

5. Composition selon l'une quelconque des revendications 1 et 2, dans laquelle lesdits antigènes tumoraux comprennent un ou plusieurs néoantigènes.

6. Composition selon l'une quelconque des revendications 1 à 5, dans laquelle ladite ou lesdites cellules tumorales comprennent des cellules autologues.

7. Composition selon l'une quelconque des revendications 1 à 5, dans laquelle ladite ou lesdites cellules tumorales comprennent des cellules allogéniques.

8. Composition selon l'une quelconque des revendications 1 à 7 pour une utilisation dans une méthode de traitement d'un cancer chez un mammifère, ladite méthode comprenant l'administration de ladite composition audit mammifère.

9. Composition selon la revendication 8, dans laquelle ladite ou lesdites cellules tumorales sont obtenues à partir d'un mammifère à traiter.

10. Composition selon la revendication 8, dans laquelle ladite ou lesdites cellules tumorales sont obtenues à partir d'un mammifère donneur.

11. Composition selon l'une quelconque des revendications 8 à 10, ledit mammifère étant un humain.

12. Composition selon l'une quelconque des revendications 8 à 11, le cancer étant choisi parmi un gliome, un mélanome, un cancer de la prostate et un cancer du poumon.
